# EUROPEAN PATENT APPLICATION

(11) **EP 3 263 562 A1**
(43) Date of publication of application: **03.01.2018**
(21) Application number: 17186299.8
(22) Date of filing: 23.12.2010
(51) Int. Cl.: C07D 257/10, A61K 31/365, A61P 35/00, A61K 31/00, A61K 31/60, C07D 259/00

(54) **RADIOPHARMACEUTICAL COMPLEXES**

(30) Priority: 24.12.2009 US 290155 P
(62) Divisional of application: 10840184.5
(71) Applicant: Lumiphore, Inc., Richmond, CA 94804 (US)
(72) Inventor: XU, Jide, Berkeley, California 94703 (US); BUTLIN, Nathaniel, G., San Francisco, California 94122 (US); MAGDA, Darren, San Leandro, California 94577 (US)
(74) Representative: HGF Limited

(57) **Abstract**

The invention provides compounds such as chelating agents useful in chelating metal ions, particularly radionuclides, to provide metal ion complexes. The invention also provides methods of using the compounds and complexes of the invention, such as in therapeutic and diagnostic applications.

SEQUENCE LISTING

<110>  Lumiphore, Inc.

<120>  RADIOPHARMACEUTICAL COMPLEXES

<130>  P203584EPD1

<150>  US 61/290,155
<151>  2009-12-24

<150>  WO2011/079291
<151>  2010-12-23

<160>  2

<170>  PatentIn version 3.5

<210>  1
<211>  18
<212>  DNA
<213>  Artificial Sequence

<220>
<223>  A DNA 18-base oligonucleotide

<400>  1
aaggtcatcc atgacaac                                                    18


<210>  2
<211>  17
<212>  DNA
<213>  Artificial Sequence

<220>
<223>  Polynucleotide for conjugation to Lumi4®-N-hydroxysuccinimide

<400>  2
agtacgacaa ccacatc                                                     17

## Description

### CROSS REFERENCE TO RELATED APPLICATIONS

This application claims under 35 USC 119(e) the benefit of US Application 61/290,155, filed December 24, 2009, which is incorporated by reference in its entirety.

### TECHNICAL FIELD

The invention relates to chemical compounds and complexes that can be used in therapeutic and diagnostic applications.

### BACKGROUND

Numerous chelating agents for binding metal ions are known in the art. These chelating agents include catechols, hydroxypyridinones, hydroxyphthalamides, and salicylamides bound together via a linking structure. See U.S. Patent Nos. 4,181,654; 4,309,305; 4,442,305; 4,543,213; 4,698,431; 4,939,254; 5,010,191; 5,049,280; 5,624,901; 5,892,029; 6,406,297; 6,515,113; 6,846,915; 6,864,103; 7,018,850; 7,404,912; and 7,442,558; US/2008/0213917; WO/2008/008797; and US/2008/0213780. Previous applications of these chelating agents have been directed to, for example, the removal of certain metal ions from tissue, the use of MRI-active lanthanide ions such as gadolinium (III) in diagnostics and the use of luminescent lanthanide ions in various binding assays.

Lanthanide complexes used for medical diagnosis must be kinetically stable because neither product of dissociation from the Ln³⁺ aqua ions or the ligands may not be well tolerated in vivo, which leads to toxicity. Therefore, high thermodynamic stability and kinetic inertness is an important criterion. This has led to the design of complexes with macrocycles such as DTPA cyclic derivatives and DOTA /DOTA derivatives, which have been extensively studied as in vivo agents. Several groups have demonstrated that dissociation of [Ln(DOTA)]⁻ and Ln(DOTA) derivatives takes place by a proton-assisted pathway and that endogenous metal ions such as Cu²⁺ or Zn²⁺ have little effect on the dissociation rate allowing it to be advantageous for some in vivo applications such as magnetic resonance imaging contrast agents.

Although, the lanthanide(III) DOTA complexes are amongst the most thermodynamically stable and kinetically inert complexes known to date, the slow kinetics of formation of the complex is problematic in applications where fast kinetics is required. For example, in alpha-particle anticancer therapy, fast and strong complexation to the alpha emitter is essential. Hence, in the design of chelates for alpha cancer therapy agents, key issues such as fast chelation, delivery and excretion as well as high affinity and low toxicity must be considered.

Whilst kinetic inertness and high structural pre-organization is desired for complex stability, ligand designs will have to be shifted away from the more stable macrocycles to compensate for a much faster kinetic interchange. The literature has shown that the complexation of metals involving macrocyclic ligands is usually several orders of magnitude slower than that with linear polyamino-polycarboxylate type ligands. The slower kinetics of association and dissociation of macrocycles especially in the case of DOTA and DOTA derivative complexes is mostly related to their high stability and high structural pre-organization. In DOTA complexes, the kinetic analysis of complex formation has shown that these systems takes place in several discrete steps where a stable intermediate is considered to be formed. The Ln³⁺ ion is positioned "out of cage" and one or two of the macrocyclic amino groups remains protonated, which has been detected by several spectroscopic techniques. Subsequent slow base-catalyzed deprotonation of those amines and rearrangement has led to the formation of a final [Ln(DOTA)]⁻ complex where the Ln³⁺ ion is trapped "in cage". Evidence for similar stable intermediate has been reported during formation of other DOTA derivatives as well.

There remains a need for novel chelating agents and complexes that are particularly suited for use as radiopharmaceuticals. Such chelating agents and complexes and methods of their use are provided by the present invention.

### SUMMARY OF INVENTION

The present invention provides a class of chelating agents of use to chelate metal ions, e.g. radionuclides, and are particularly useful for forming complexes with therapeutic or diagnostic value. Useful chelators comprise chelating moieties selected in any combination from 1,2-hydroxypyridinone-based ligands ("1,2-HOPO"), maltol derivatives, hydroxypyrimidinone (HOPY) derivatives, hydroxy-iso-phthalic acid derivatives, catecholic acid derivatives, terephthalic acid derivatives (e.g., terephthalamidyl, TAM) and salicylic acid derivatives. Combinations of these moieties can be incorporated into a single ligand in which the subunits are linked by one or more scaffold moieties, e.g., tris(2-aminoethyl)amine (TREN) and, preferably tetrapodal topology scaffolds, such as H22. Exemplary chelators also comprise a functionalized linker that can be used to attach a targeting moiety to the chelators. Accordingly, the invention provides a chelator linked to a targeting moiety. The targeting moiety can be any moiety with a particular affinity for some locus within an animal, cell or investigated system. In this way, the chelators and complexes provided herein can be directed to a site of interest for therapeutic or diagnostic purposes.

One advantage of the present complexes is that they exhibit high stability in solution. As such the complexes of the invention can be used as probes, such as in microscopy, enzymology, clinical chemistry, molecular biology and medicine. The compounds of the invention are also useful as therapeutic agents and as diagnostic agents in imaging methods.

While chelating agents may be shown in their complexed form (e.g., complexed with M⁺³), the structures represented by the formulae shown herein are not limited to metal ion complexes, which are merely one form of the chelating agent. The formulae are equally representative of the uncomplexed chelating agents. However, in some instances, a complex may have a specific property imparted to the complex by chelation of the metal ion (e.g., radioactivity).

### BRIEF DESCRIPTION OF DRAWINGS

Figure 1 shows a scheme for making an open chelator comprising reactive functional groups.
Figure 2 shows a scheme for making a macrocycle.
Figure 3 shows a scheme for making an open chelator comprising reactive functional groups.
Figure 4 shows a scheme for making a macrocycle.
Figure 5 shows a scheme for conjugating a targeting moiety to a chelator.
Figure 6 shows crystal structures for Me4BH(2,2)IAM complexed with an ion.

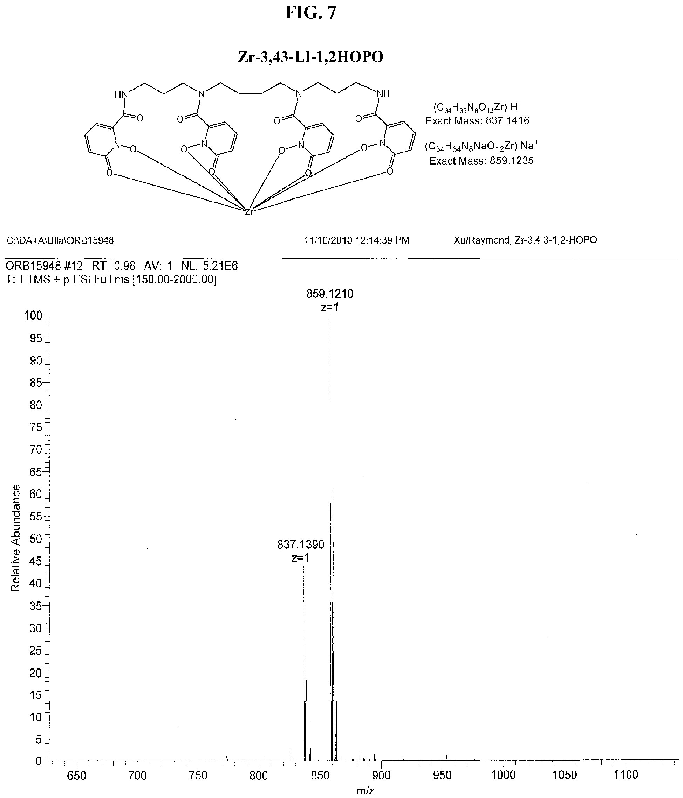

Figure 7 shows mass spectrometry data for a Zr-3,4,3-LI-1,2-HOPO complex.

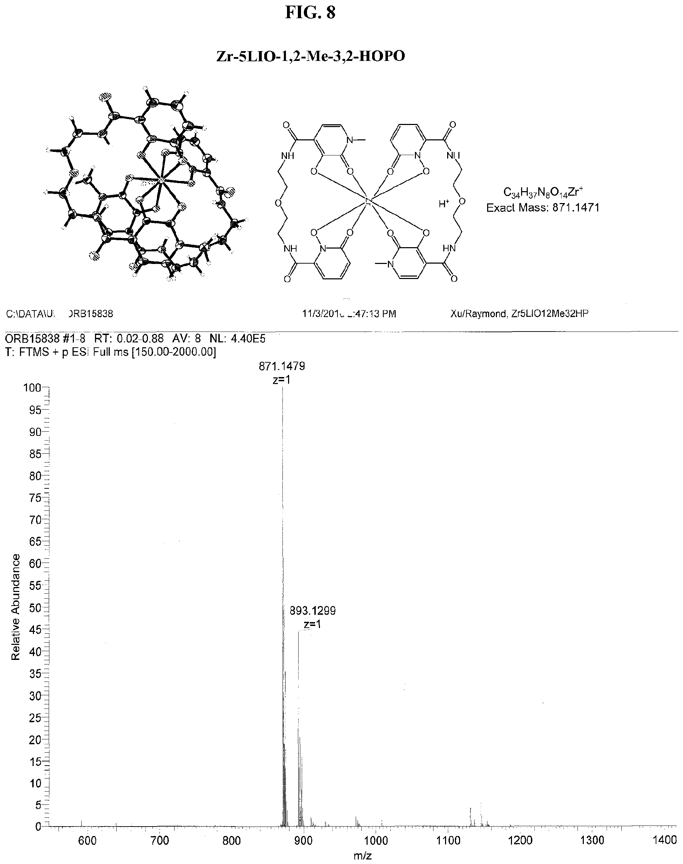

Figure 8 shows a crystal structure and mass spectrometry data for a Zr-5LIO-1,2-Me-3,2-HOPO complex.

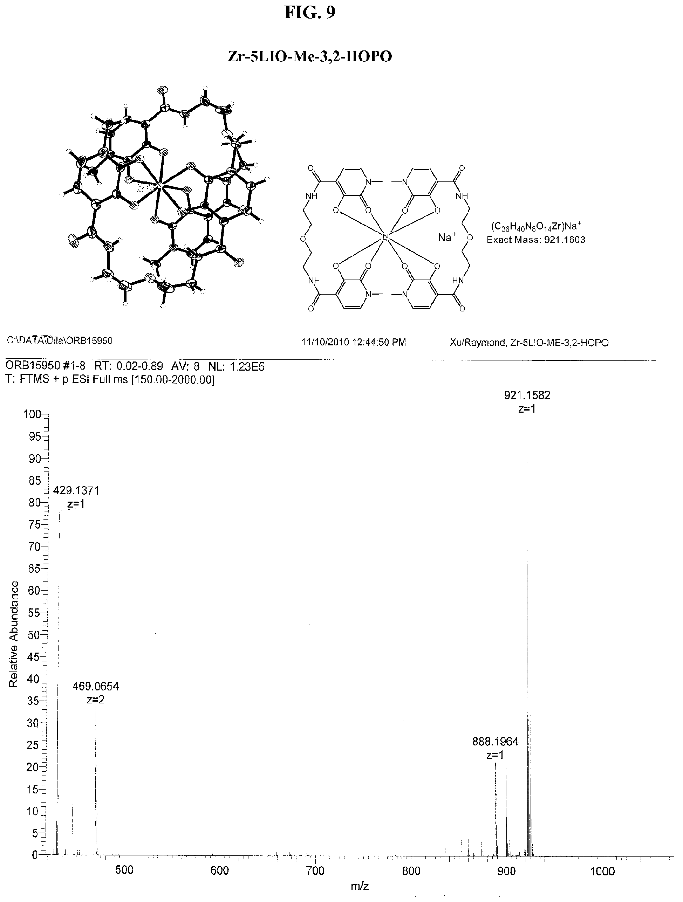

Figure 9 shows a crystal structure and mass spectrometry data for a Zr-5LIO-Me-3,2-HOPO complex.

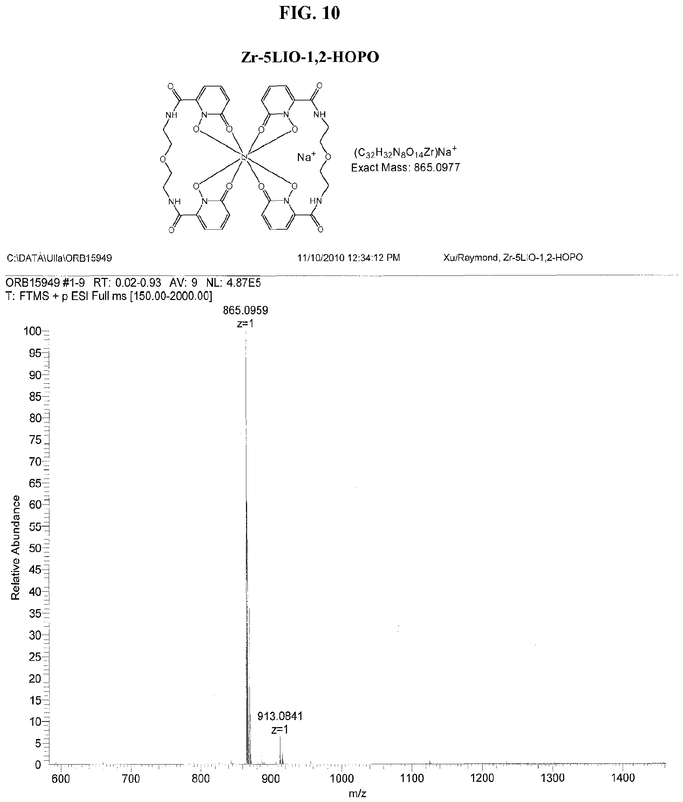

Figure 10 shows mass spectrometry data for a Zr-5LIO-1,2-HOPO complex.

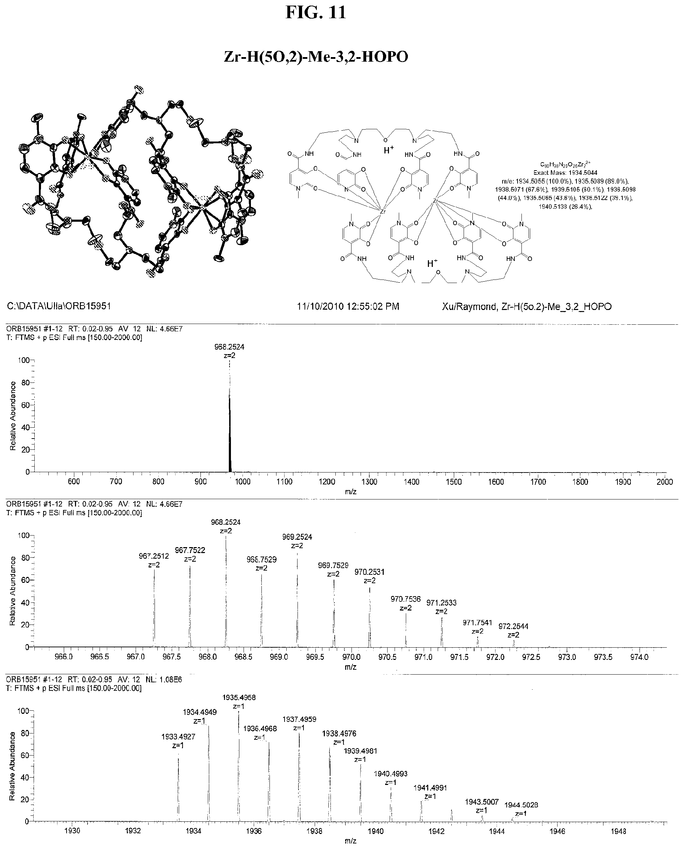

Figure 11 shows a crystal structure and mass spectrometry data for a Zr-H(5O,2)-Me-3,2-HOPO complex

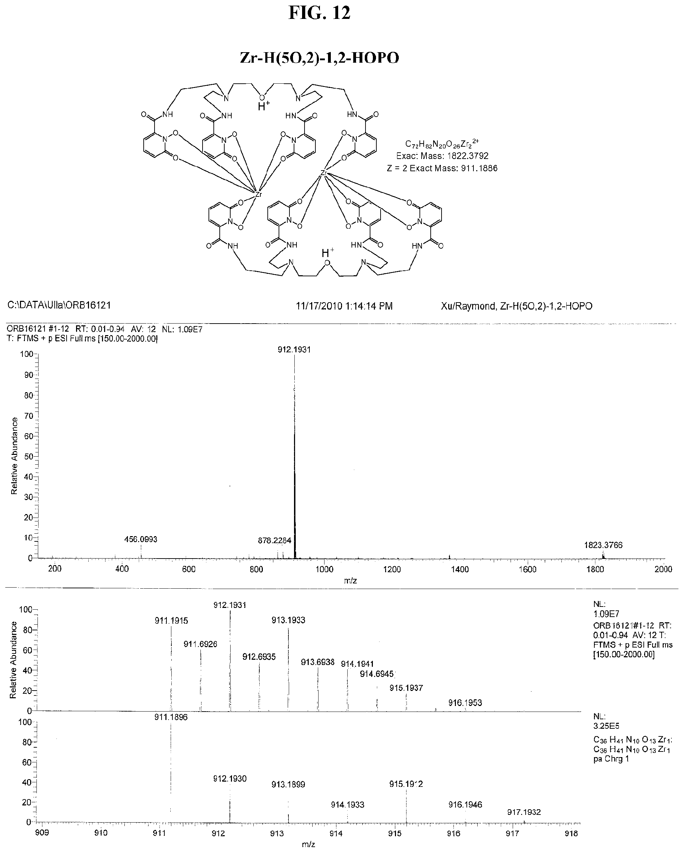

Figure 12 shows mass spectrometry data for a Zr-H(5O,2)-1,2-HOPO complex.
Figure 13, Figure 14 and Figure 15 show crystal structures for a Ce(IV)-H(2,2)-1,2-HOPO complex.
Figure 16 shows mass spectrometry data for a Dy-Lumi4® complex.
Figure 17 shows mass spectrometry data for a Yb-Lumi4® complex.
Figure 18 shows kinetic association luminescent measurements for determining the rate of complexation of Tb-Lumi4^{®}-NH₂.
Figure 19 shows electrophoresis of oligonucleotide 1 and oligonucleotide-Lumi4 conjugate 2 in the presence and absence of pretreatment with metal cations.
Figure 20 shows a scheme for conjugating Lumi4®-N-hydroxysuccinimide to a polynucleotide.

### DESCRIPTION OF EMBODIMENTS

### Definitions

Where substituent groups are specified by their conventional chemical formulae, written from left to right, they optionally equally encompass the chemically identical substituents, which would result from writing the structure from right to left, *e.g.,* -CH₂O- is intended to also recite -OCH₂-.

The term "alkyl", by itself or as part of another substituent, means a straight or branched chain hydrocarbon, which may be fully saturated, mono- or polyunsaturated and includes mono-, di- and multivalent radicals. Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds (i.e., alkenyl and alkynyl moieties). Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl" can refer to "alkylene", which by itself or as part of another substituent means a divalent radical derived from an alkane, as exemplified, but not limited, by -CH₂CH₂CH₂CH₂-. Typically, an alkyl (or alkylene) group will have from 1 to 30 carbon atoms. A "lower alkyl" or "lower alkylene" is a shorter chain alkyl or alkylene group, generally having eight or fewer carbon atoms. In some embodiments, alkyl refers to an alkyl or combination of alkyls selected from C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉ and C₃₀ alkyl. In some embodiments, alkyl refers to C₁-C₂₅ alkyl. In some embodiments, alkyl refers to C₁-C₂₀ alkyl. In some embodiments, alkyl refers to C₁-C₁₅ alkyl. In some embodiments, alkyl refers to C₁-C₁₀ alkyl. In some embodiments, alkyl refers to C₁-C₆ alkyl.

The term "heteroalkyl," by itself or in combination with another term, means an alkyl in which one or more carbons are replaced with one or more heteroatoms selected from the group consisting of O, N, Si and S, (preferably O, N and S), wherein the nitrogen and sulfur atoms may optionally be oxidized and the nitrogen heteroatom may optionally be quaternized. The heteroatoms O, N, Si and S may be placed at any interior position of the heteroalkyl group or at the position at which the alkyl group is attached to the remainder of the molecule. In some embodiments, depending on whether a heteroatom terminates a chain or is in an interior position, the heteroatom may be bonded to one or more H or substituents such as (C₁, C₂, C₃, C₄, C₅ or C₆) alkyl according to the valence of the heteroatom. Examples of heteroalkyl groups include, but are not limited to, -CH₂-CH₂-O-CH₃, -CH₂-CH₂-NH-CH₃, -CH₂-CH₂-N(CH₃)-CH₃, - CH₂-S-CH₂-CH₃, -CH₂-CH₂,-S(O)-CH₃, -CH₂-CH₂-S(O)₂-CH₃, -CH=CH-O-CH₃, - Si(CH₃)₃, -CH₂-CH=N-OCH₃, and -CH=CH-N(CH₃)-CH₃. No more than two heteroatoms may be consecutive, as in, for example, -CH₂-NH-OCH₃ and -CH₂-O-Si(CH₃)₃, and in some instances, this may place a limit on the number of heteroatom substitutions. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. The designated number of carbons in heteroforms of alkyl, alkenyl and alkynyl includes the heteroatom count. For example, a (C₁, C₂, C₃, C₄, C₅ or C₆) heteroalkyl will contain, respectively, 1, 2, 3, 4, 5 or 6 atoms selected from C, N, O, Si and S such that the heteroalkyl contains at least one C atom and at least one heteroatom, for example 1-5 C and 1 N or 1-4 C and 2 N. Further, a heteroalkyl may also contain one or more carbonyl groups. In some embodiments, a heteroalkyl is any C₂-C₃₀ alkyl, C₂-C₂₅ alkyl, C₂-C₂₀ alkyl, C₂-C₁₅ alkyl, C₂-C₁₀ alkyl or C₂-C₆ alkyl in any of which one or more carbons are replaced by one or more heteroatoms selected from O, N, Si and S (or from O, N and S). In some embodiments, each of 1, 2, 3, 4 or 5 carbons is replaced with a heteroatom.The terms "alkoxy," "alkylamino" and "alkylthio" (or thioalkoxy) are used in their conventional sense, and refer to those alkyl and heteroalkyl groups attached to the remainder of the molecule via an oxygen atom, a nitrogen atom (e.g., an amine group), or a sulfur atom, respectively.

The terms "cycloalkyl" and "heterocycloalkyl", by themselves or in combination with other terms, refer to cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1 -(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The term "aryl" means a polyunsaturated, aromatic substituent that can be a single ring or optionally multiple rings (preferably 1, 2 or 3 rings) that are fused together or linked covalently. In some embodiments, aryl is a 3, 4, 5, 6, 7 or 8 membered ring, which is optionally fused to one or two other 3, 4, 5, 6, 7 or 8 membered rings. The term "heteroaryl" refers to aryl groups (or rings) that contain 1, 2, 3 or 4 heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl.

In some embodiments, any of alkyl, heteroalkyl, cycloalkyl, heterocycloalkyl, aryl and heteroaryl is optionally substituted. That is, in some embodiments, any of these groups is substituted or unsubstituted. In some embodiments, substituents for each type of radical are selected from those provided below.

Substituents for the alkyl, heteroalkyl, cycloalkyl and heterocycloalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents". In some embodiments, an alkyl group substituent is selected from -halogen, -OR', =O, =NR', =N-OR', -NR'R", -SR', -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -CN and -NO₂ in a number ranging from zero to (2m'+1), where m' is the total number of carbon atoms in such radical. In one embodiment, R', R", R"' and R"" are each independently selected from hydrogen, alkyl (e.g., C₁, C₂, C₃, C₄, C₅ and C₆ alkyl). In one embodiment, R', R", R"' and R"" each independently refer to hydrogen, substituted or unsubstituted heteroalkyl, substituted or unsubstituted aryl, *e.g.,* aryl substituted with 1-3 halogens, substituted or unsubstituted alkyl, alkoxy or thioalkoxy groups, or arylalkyl groups. In one embodiment, R', R", R"' and R"" are each independently selected from hydrogen, alkyl, heteroalkyl, cycloalkyl, heterocycloalkyl, aryl, heteroaryl, alkoxy, thioalkoxy groups, and arylalkyl. When R' and R" are attached to the same nitrogen atom, they can be combined with the nitrogen atom to form a 5-, 6-, or 7-membered ring. For example, -NR'R" can include 1-pyrrolidinyl and 4-morpholinyl. In some embodiments, an alkyl group substituent is selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl.

Similar to the substituents described for the alkyl radical, substituents for the aryl and heteroaryl groups are generically referred to as "aryl group substituents". In some embodiments, an aryl group substituent is selected from -halogen, -OR', =O, =NR', =N-OR', -NR'R", -SR', -SiR'R"R"', -OC(O)R', -C(O)R', -CO₂R', -CONR'R", - OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -CN and -NO₂, -R', -N₃, -CH(Ph)₂, fluoro(C₁-C₄)alkoxy, and fluoro(C₁-C₄)alkyl, in a number ranging from zero to the total number of open valences on the aromatic ring system. In some embodiments, R', R", R"' and R"" are independently selected from hydrogen and alkyl (e.g., C₁, C₂, C₃, C₄, C₅ and C₆ alkyl). In some embodiments, R', R", R"' and R"" are independently selected from hydrogen, substituted or unsubstituted alkyl, substituted or unsubstituted heteroalkyl, substituted or unsubstituted aryl and substituted or unsubstituted heteroaryl. In some embodiments, R', R", R"' and R"" are independently selected from hydrogen, alkyl, heteroalkyl, aryl and heteroaryl. In some embodiments, an aryl group substituent is selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl.

Two of the substituents on adjacent atoms of the aryl or heteroaryl ring may optionally be replaced with a substituent of the formula -T-C(O)-(CRR')_{q}-U-, wherein T and U are independently -NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X-(CR"R"')_{d}-, where s and d are independently integers of from 0 to 3, and X is -O-, -NR'-, -S-, -S(O)-, -S(O)₂-, or - S(O)₂NR'-. The substituents R, R', R" and R'" are preferably independently selected from hydrogen or substituted or unsubstituted (C₁-C₆)alkyl.

The term "acyl" refers to a species that includes the moiety -C(O)R, where R has the meaning defined herein. Exemplary species for R include H, halogen, substituted or unsubstituted alkyl, substituted or unsubstituted aryl, substituted or unsubstituted heteroaryl, and substituted or unsubstituted heterocycloalkyl. In some embodiments, R is selected from H and (C₁-C₆)alkyl.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like. In some embodiments, halogen refers to an atom selected from F, Cl and Br.

The term "heteroatom" includes oxygen (O), nitrogen (N), sulfur (S) and silicon (Si). In some embodiments, a heteroatom is selected from N and S. In some embodiments, the heteroatom is O.

Unless otherwise specified, the symbol "R" is a general abbreviation that represents a substituent group that is selected from acyl, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound includes more than one R, R', R", R'" and R"" group, they are each independently selected.

For groups with solvent exchangeable protons, the ionized form is equally contemplated. For example, -COOH also refers to -COO⁻ and -OH also refers to -O⁻.

Any of the compounds disclosed herein can be made into a pharmaceutically acceptable salt. The term "pharmaceutically acceptable salts" includes salts of compounds that are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., Journal of Pharmaceutical Science, 66: 1-19 (1977)). Certain specific compounds of the present invention contain both basic and acidic functionalities that allow the compounds to be converted into either base or acid addition salts. The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides any of the compounds disclosed herein in a prodrug form. Prodrugs of the compounds described herein are those compounds that readily undergo chemical changes under physiological conditions to provide the compounds of the present invention.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

### Compositions

The invention provides numerous chelators and metal ion complexes thereof. Generally, a chelator comprises a plurality of chelating agents that are linked together by way of one or more scaffold moieties. Chelating moieties bound together by one scaffold moiety can be referred to as open chelators, while those bound together by two scaffold moieties such that at least one closed ring is formed can be referred to as closed chelators, macrocycles or macrocyclic chelators.

Many different types of chelating moieties can be used in the chelating agents and complexes disclosed herein. For example, 1,2-HOPO is a useful chelating moiety. Th(IV)-(1,2-HOPO)₄ crystals form in water at pH 7. HOPO units are more acidic than catecholates and hydroxamic acids. They are powerful, selective chelators for "hard" metal ions, ionized at physiological pH. Multidentate agents should achieve 8-, 9- or higher coordination to a tetravalent actinide An(IV) and should stably bind An(IV) in physiological pH solution. Successful removal of Pu(IV) from mice by linear 1,2-HOPO ligands indicated 8-coordination with 3,4,3-LI(1,2-HOPO).

An exemplary chelator is octadentate 3,4,3-LI(1,2-HOPO), which is thermodynamically, structurally and kinetically competitive for Anⁿ⁺ and AnO₂ⁿ⁺ with transferrin, other plasma proteins, carbonate, ferritin, bone mineral. Injected, infused or oral 3,4,3-LI(1,2-HOPO) removes more injected or inhaled Pu(IV) as well as Am(III) from rodents than CaNa₃-DTPA. Injected ip or infiltrated into a wound, 3,4, 3-LI(1,2-HOPO) is much more effective for removing Th(IV), Pu(IV) or Am(III) from the wound site and body than CaNa₃-DTPA. Injected or oral Fe(III)-3,4,3-LI(1,2-HOPO) is almost as effective for Pu(IV) removal as the native ligand. This agent effectively competes for Pu(IV) and Am(III) sorbed to bone mineral, and is effective at very low dosage (0.01-0.1 µmol/Kg). It forms excretable actinide chelates at physiological pH and has useful oral activity and an acceptably low toxicity at effective dosage.

Based on the well defined background on the kinetic and in vivo properties of the open chain octadentate ligand DTPA and their derivatives, octadentate chelates disclosed herein are an ideal design as an anticancer chelator for thorium, specifically those isotopes that decay via alpha-emission. These are also open chain, linear chelators designed to give faster kinetics with strong binding affinity to the metal. Low toxicity is another essential requirement as well which has been shown by our past work to be influenced by the type of chelating unit, ligand multidenticity, and topology in the ligand design.

There are several factors to be considered in the design for an alpha chelating agent for anticancer therapy. Some of the key issues apart from the kinetics will be the high affinity for the target metal (Th) which at the same time needs to have a low exchange rate for other biologically significant metal ions. So, in our ligand design, the electronic properties of the target metal and ligand are considered and matched. The chelate should also be able to assume the appropriate coordination cavity size and geometry for the desired metal. In this case, Th, an actinide ion, is a "hard" cation and has large charge-to-radius ratios. Hence, Th prefers "hard" oxygen and negatively charged oxygen donors. A coordination number of 8 or greater is generally preferred by actinide ions as they have a tendency to form stable complexes with ligands of high denticity; however, the selectivity towards the binding of the thorium will be determined by our design of the chelating unit. The effective but nonselective aminocarboxylic acid ligands such as DTPA can deplete essential biological metal ions from patients, thus causing serious health problems. Selecting the correct type of chelating unit, therefore, is an important factor in achieving high selectivity toward the specific metal ion.

A chelator can comprise numerous chelating moieties. Particularly useful chelators contain a number of chelating moieties sufficient to provide, for example, 6, 8 or 10 heteroatoms such as oxygen that coordinate with a metal ion to form a complex. The heteroatoms such as oxygen provide electron density for forming coordinate bonds with a positively charged ion, and such heteroatoms can thus be considered "donors". In some embodiments, the plurality of chelating moieties of a chelator comprises a plurality of oxygen donors and a radionuclide is chelated to the chelator via at least one of the oxygen donors. In some embodiments, a chelator comprises a plurality of oxygen donors and a radionuclide is chelated to the chelator via a plurality or all of the oxygen donors.

Accordingly, in one aspect, the invention provides a complex comprising (a) a radionuclide and (b) a macrocycle comprising (i) a plurality of chelating moieties, (ii) a linker, (iii) a first scaffold moiety and (iv) a second scaffold moiety, wherein each of the chelating moieties is attached to the first scaffold moiety and the second scaffold moiety. In some embodiments, the macrocycle comprises 3, 4 or 5 chelating moieties. In one aspect, the invention provides a complex comprising (a) a radionuclide and (b) a macrocycle comprising (i) a plurality of chelating moieties, (ii) a first scaffold moiety and (iii) a second scaffold moiety, wherein each of the chelating moieties is attached to the first scaffold moiety and the second scaffold moiety.

Also provided herein are uncomplexed forms of any chelator described herein. Thus, in one aspect, the invention provides a macrocycle comprising (i) a plurality of chelating moieties, (ii) a linker, (iii) a first scaffold moiety and (iv) a second scaffold moiety, wherein each of the chelating moieties is attached to the first scaffold moiety and the second scaffold moiety.

In exemplary embodiments, a macrocycle comprises chelating moieties independently selected from 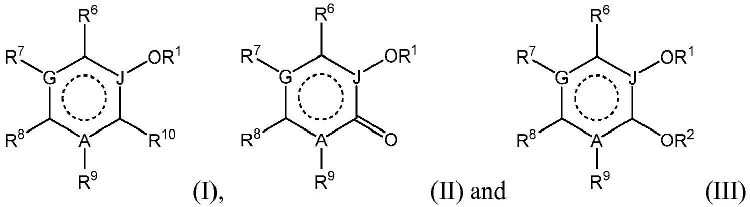 wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸ and -NO₂; R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl; R¹⁷ and R¹⁸, together with the atoms to which they are attached, are optionally joined to form a 5-, 6- or 7-membered ring; at least two of R⁶, R⁷, R⁸, R⁹ and R¹⁰ are optionally joined to form a ring system which is a member selected from substituted or unsubstituted cycloalkyl, substituted or unsubstituted heterocycloalkyl, substituted or unsubstituted aryl and substituted or unsubstituted heteroaryl; R¹ and R² are each independently selected from H and a negative charge; A, G and J are independently selected from carbon and nitrogen; and wherein one of R⁶ and R⁹ in (II) or (III) or one of R⁶ and R¹⁰ in (I) comprises a bond to the first scaffold moiety, with the other of R⁶ and R⁹ in (II) or (III) and the other of R⁶ and R¹⁰ in (I) comprising a bond to the second scaffold moiety.

In some embodiments, R¹ and R² is independently selected from H, an enzymatically labile group, a hydrolytically labile group, a metabolically labile group, a photolytic group.

In some embodiments, R⁷ and R⁸ are selected from H, halogen, alkyl, haloalkyl, heteroalkyl, aryl, heteroaryl, -C(O)R¹⁷, -SO₂NR¹⁷R¹⁸ -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl. In some embodiments, R⁷ and R⁸ are selected from H and (C₁, C₂, C₃, C₄, C₅ or C₆) alkyl. In exemplary embodiments, R⁷ and R⁸ are H.

In exemplary embodiments, R¹⁷ and R¹⁸ are selected from H and (C₁, C₂, C₃, C₄, C₅ or C₆) alkyl.

In some embodiments, if one of the chelating moieties has the structure 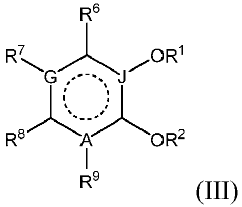 then all of the chelating moieties are not the same. In some embodiments, the macrocycle is not BH(2,2)CAM as known in the art. In exemplary embodiments, not all of the chelating moieties have the structure 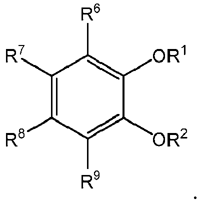 In some embodiments, the chelating moieties all have the structure

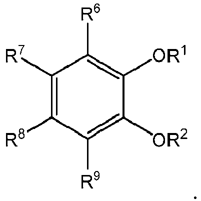

In some embodiments, the chelating moieties all have the structure

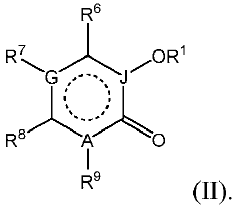

In exemplary embodiments, the chelating moieties all have the structure

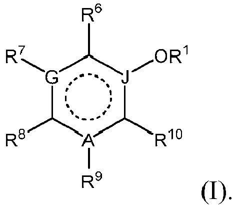

In some embodiments, R⁹ of 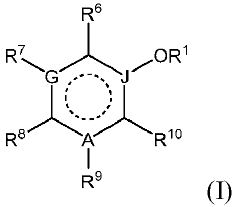 is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁸SO₂R¹⁸, wherein R¹⁷ and R¹⁸ are selected from H and alkyl. In some embodiments, R⁹ is selected from H and (C₁, C₂, C₃, C₄, C₅ or C₆) alkyl. In exemplary embodiments, R⁹ is H.

In exemplary embodiments, in structure (I), A, G and J are carbon. In some embodiments, in structure (II), A is nitrogen and G and J are carbon. In some embodiments, in structure (II), J is nitrogen and A and G are carbon. In some embodiments, in structure (III), A, G and J are carbon.

In one aspect, the invention provides a complex comprising (a) a radionuclide and (b) a macrocycle comprising (i) a plurality of chelating moieties having the structure 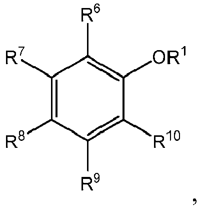 (ii) a linker, (iii) a first scaffold moiety and (iv) a second scaffold moiety, wherein each of the chelating moieties is attached to the first scaffold moiety and the second scaffold moiety. R¹, R⁶, R⁷, R⁸, R⁹ and R¹⁰ are as described herein. In exemplary embodiments, R⁷, R⁸ and R⁹ are H. In one aspect, the invention provides the macrocycle itself, that is, the complex in the absence of the radionuclide.

In one aspect, the invention provides a complex comprising (a) a radionuclide and (b) a macrocycle comprising (i) a plurality of chelating moieties having the structure 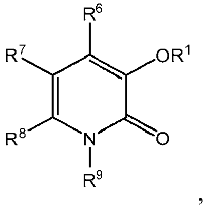 (ii) a linker, (iii) a first scaffold moiety and (iv) a second scaffold moiety, wherein each of the chelating moieties is attached to the first scaffold moiety and the second scaffold moiety. R¹, R⁶, R⁷, R⁸ and R⁹ are as described herein. In exemplary embodiments, R⁷ and R⁸ are H. In one aspect, the invention provides the macrocycle itself, that is, the complex in the absence of the radionuclide.

In one aspect, the invention provides a complex comprising (a) a radionuclide and (b) a macrocycle comprising (i) a plurality of chelating moieties having the structure 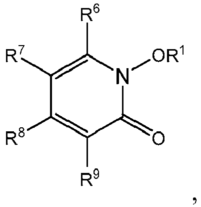 (ii) a linker, (iii) a first scaffold moiety and (iv) a second scaffold moiety, wherein each of the chelating moieties is attached to the first scaffold moiety and the second scaffold moiety. R¹, R⁶, R⁷, R⁸ and R⁹ are as described herein. In exemplary embodiments, R⁷ and R⁸ are H. In one aspect, the invention provides the macrocycle itself, that is, the complex in the absence of the radionuclide.

Also provided are isomers of any macrocycle described herein, and isomers complexed to a radionuclide. In one embodiment, a compound has a structure according to Formula (Ia): 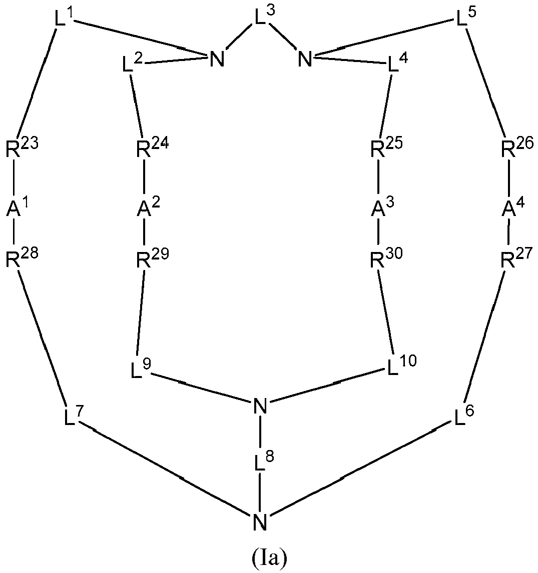 L³ comprises -(CH₂CH₂O)ₘR³¹- and L⁸ comprises -(CH₂CH₂O)ₙR³²- wherein m and n are integers independently selected from 0, 1, 2, 3, 4, 5, 6, 7, 8 and 9; A¹, A², A³, A⁴, L¹, L², L⁴ L⁵, L⁶, L⁷, L⁹, L¹⁰, R³¹ and R³² are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; R²³, R²⁴, R²⁵, R²⁶, R²⁷, R²⁸, R²⁹ and R³⁰ are independently selected from a bond, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; at least one of A¹, A², A³ and A⁴ is selected from 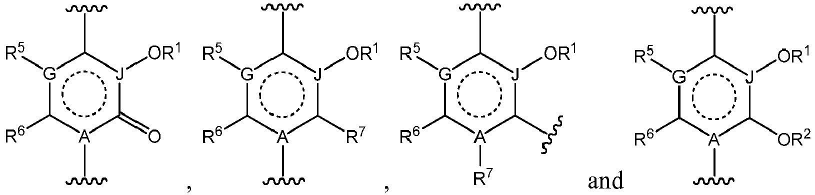 wherein A, G and J are atoms independently selected from carbon and nitrogen; each R¹ and R² is independently selected from H, an enzymatically labile group, a hydrolytically labile group, a metabolically labile group, a photolytic group and a single negative charge; each R⁵, R⁶ and R⁷ is independently selected from H, substituted or unsubstituted alkyl, substituted or unsubstituted heteroalkyl, halogen, CN, CF₃, acyl, -SO₂NR¹⁷R¹⁸, -NR¹⁷R¹⁸, -OR¹⁷, -S(O)₂R¹⁷,-COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, and -NO₂, R⁵ and R⁶ are optionally joined to form a ring system which is a member selected from substituted or unsubstituted cycloalkyl, substituted or unsubstituted heterocycloalkyl, substituted or unsubstituted aryl and substituted or unsubstituted heteroaryl; and R¹⁷ and R¹⁸ are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; and R¹⁷ and R¹⁸, together with the atoms to which they are attached, are optionally joined to form a 5-, 6- or 7-membered ring. Other suitable substituents from corresponding chelating moieties and scaffolds disclosed herein can be used.

In one aspect, the invention provides a complex comprising (a) a radionuclide and (b) a chelator comprising (i) a plurality of chelating moieties and (ii) a first scaffold moiety, wherein each of the chelating moieties is attached to the first scaffold moiety and wherein each of the chelating moieties has a structure independently selected from 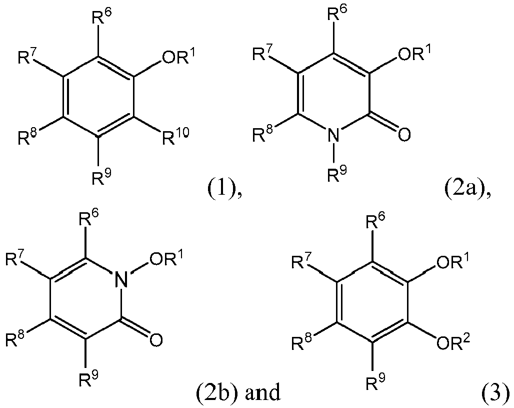 wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸ and -NO₂; R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; R¹⁷ and R¹⁸, together with the atoms to which they are attached, are optionally joined to form a 5-, 6- or 7-membered ring; at least two of R⁶, R⁷, R⁸, R⁹ and R¹⁰ are optionally joined to form a ring system selected from substituted or unsubstituted cycloalkyl, substituted or unsubstituted heterocycloalkyl, substituted or unsubstituted aryl and substituted or unsubstituted heteroaryl; R¹ and R² are each independently selected from H and a negative charge; one of R⁶ and R⁹ in (2a), (2b) and (3) and one of R⁶ and R¹⁰ in (1) comprises a bond to the first scaffold moiety, wherein at least one chelating moiety has the structure

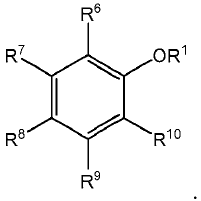

In some embodiments, R¹ and R² is independently selected from H, an enzymatically labile group, a hydrolytically labile group, a metabolically labile group, a photolytic group.

In some embodiments, R⁷ and R⁸ are selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl. In some embodiments, R⁷ and R⁸ are selected from H and (C₁, C₂, C₃, C₄, C₅ or C₆) alkyl. In exemplary embodiments, R⁷ and R⁸ are H.

In exemplary embodiments, R¹⁷ and R¹⁸ are selected from H and (C₁, C₂, C₃, C₄, C₅ or C₆) alkyl.

In exemplary embodiments, all the chelating moieties have the structure

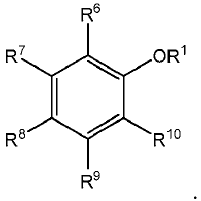

In some embodiments, if R⁶ is attached to the first scaffold moiety, then R⁹ in (2a), (2b) and (3) or R¹⁰ in (1) is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl; and if R⁹ in (2a), (2b) and (3) or R¹⁰ in (1) is attached to the first scaffold moiety, then R⁶ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl. In some embodiments, if R⁶ is attached to the first scaffold moiety, then R⁹ in (2a), (2b) and (3) or R¹⁰ in (1) is selected from H and (C₁, C₂, C₃, C₄, C₅ or C₆) alkyl; and if R⁹ in (2a), (2b) and (3) or R¹⁰ in (1) is attached to the first scaffold moiety, then R⁶ is selected from H and (C₁, C₂, C₃, C₄, C₅ or C₆) alkyl. In exemplary embodiments, if R⁶ is attached to the first scaffold moiety, then R⁹ in (2a), (2b) and (3) or R¹⁰ in (1) is H; and if R⁹ in (2a), (2b) and (3) or R¹⁰ in (1) is attached to the first scaffold moiety, then R⁶ is H.

In some embodiments, R¹⁰ is -C(O)NR¹⁷R¹⁸. In some embodiments, R¹⁷ and R¹⁸ are each independently selected from H, substituted or unsubstituted alkyl and substituted or unsubstituted heteroalkyl. In some embodiments, R¹⁷ and R¹⁸ are each independently selected from H, alkyl and heteroalkyl.

In exemplary embodiments, a complex further comprises a linker.

In one aspect, the invention provides the chelator, that is, the complex in the absence of the radionuclide.

In one aspect, the invention provides a complex comprising (a) a radionuclide and (b) a first chelator comprising (i) a plurality of chelating moieties, (ii) a linker and (iii) a first scaffold moiety, wherein each of the chelating moieties is attached to the first scaffold moiety and wherein each of the chelating moieties has a structure independently selected from: 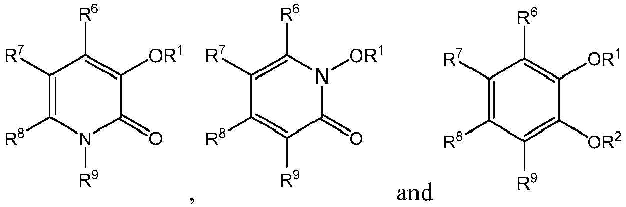 wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, -NO₂; R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl; R¹⁷ and R¹⁸, together with the atoms to which they are attached, are optionally joined to form a 5-, 6- or 7-membered ring; at least two of R⁶, R⁷, R⁸, R⁹ and R¹⁰ are optionally joined to form a ring system selected from substituted or unsubstituted cycloalkyl, substituted or unsubstituted heterocycloalkyl, substituted or unsubstituted aryl and substituted or unsubstituted heteroaryl; R¹ and R² are each independently selected from H and a negative charge; one of R⁶ and R⁹ in each chelating moiety comprises a bond to the first scaffold moiety.

In some embodiments, R¹ and R² is independently selected from H, an enzymatically labile group, a hydrolytically labile group, a metabolically labile group, a photolytic group.

In some embodiments, R⁷ and R⁸ are selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl. In some embodiments, R⁷ and R⁸ are selected from H and (C₁, C₂, C₃, C₄, C₅ or C₆) alkyl. In exemplary embodiments, R⁷ and R⁸ are H.

In exemplary embodiments, R¹⁷ and R¹⁸ are selected from H and (C₁, C₂, C₃, C₄, C₅ or C₆) alkyl.

In some embodiments, the chelating moieties are selected from

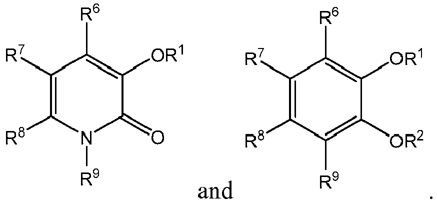

In some embodiments, the chelating moieties are selected from

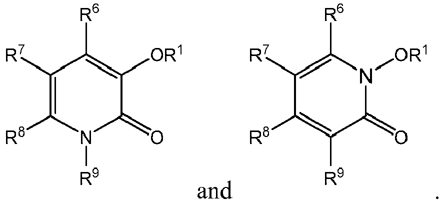

In some embodiments, if R⁶ is attached to the first scaffold moiety, then R⁹ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl; and if R⁹ is attached to the first scaffold moiety, then R⁶ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl. In some embodiments, if R⁶ is attached to the first scaffold moiety, then R⁹ is selected from H and (C₁, C₂, C₃, C₄, C₅ or C₆) alkyl; and if R⁹ is attached to the first scaffold moiety, then R⁶ is selected from H and (C₁, C₂, C₃, C₄, C₅ or C₆) alkyl. In exemplary embodiments, if R⁶ is attached to the first scaffold moiety, then R⁹ is H; and if R⁹ is attached to the first scaffold moiety, then R⁶ is H. In exemplary embodiments, if R⁶ is attached to the first scaffold moiety, then R⁹ is methyl; and if R⁹ is attached to the first scaffold moiety, then R⁶ is methyl.

In exemplary embodiments, a complex further comprises a second chelator having the same structure as the first chelator.

In exemplary embodiments, the chelating moieties are not all the same.

In one aspect, the invention provides the chelator, that is, the complex in the absence of the radionuclide.

### Scaffold Moiety

A "scaffold moiety" is any moiety useful for covalently linking two or more chelating moieties in any of the chelators (e.g., open chelators or macrocycles) disclosed herein. In exemplary embodiments, any two scaffold moieties disclosed herein are joined via a plurality of chelating moieties to form a macrocycle. In exemplary embodiments, one or more scaffold moieties of a chelator is substituted with a linker. In one embodiment, a scaffold moiety is selected from substituted or unsubstituted alkyl and substituted or unsubstituted heteroalkyl. In one embodiment, a scaffold moiety is substituted heteroalkyl. In one embodiment, a scaffold moiety is unsubstituted heteroalkyl. In one embodiment, a scaffold moiety is heteroalkyl substituted by a linker. In one embodiment, a scaffold moiety is heteroalkyl substituted by a plurality of linkers. Exemplary scaffold moieties include linear or branched ethers and amines.

Other exemplary scaffold moieties include, but are not limited to:

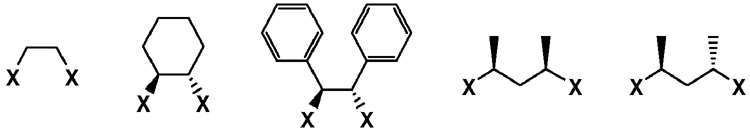

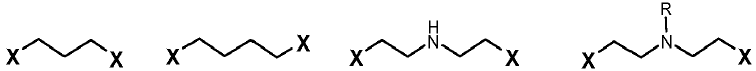

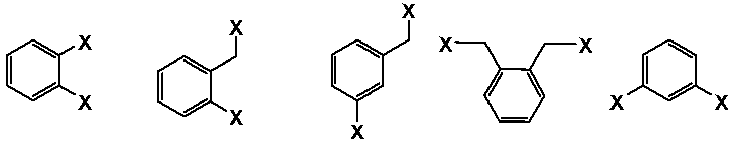

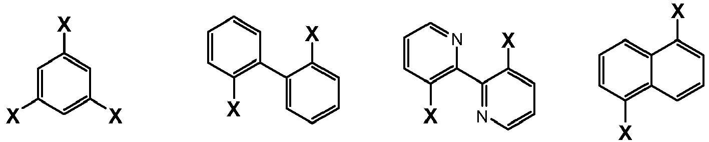

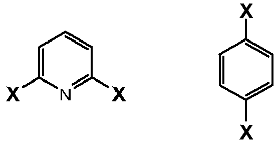

"X" represents a locus of attachment for a chelating moiety, and in exemplary embodiments includes a heteroatom such as nitrogen. Thus, in some embodiments, X is NR'R", wherein R' and R" are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, --SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, -NO₂; and R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl; wherein at least one R' or R" comprises a bond to a chelating moiety. The chelating moiety can be attached to a scaffold via any appropriate linker.

In some embodiments, a scaffold moiety is linear. One exemplary scaffold moiety is X-(CH₂)₃-X-(CH₂)₄-X-(CH₂)₃-X, which is preferably substituted (e.g. with a linker) at at least one of the alkyl moieties. That is, one exemplary scaffold moiety is spermine based. Other exemplary scaffold moieties include 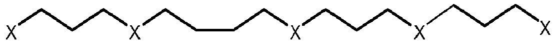 and 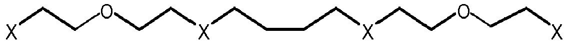 any of which is preferably substituted (e.g. with a linker) at at least one of the alkyl moieties. X is as given in the previous paragraph.

One preferred moiety for at least one of the X moieties is the 1,2-HOPO amide moiety, but those of skill in the art will appreciate that other chelating moieties in any used in any combination. In each of the scaffold structures, an aryl moiety or alkyl moiety can be substituted with one or more "aryl group substituent" or "alkyl group substituent" as defined herein.

A particularly useful scaffold moiety for any chelator described herein has the structure 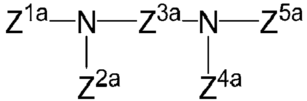 wherein Z^{1a}, Z^{2a}, Z^{3a}, Z^{4a} and Z^{5a} are selected from substituted or unsubstituted alkyl and substituted or unsubstituted heteroalkyl; and Z^{1a}, Z^{2a}, Z^{4a} and Z^{5a} comprise a bond to one of the chelating moieties.

In some embodiments, Z^{3a} is substituted or unsubstituted (C₁, C₂, C₃, C₄, C₅ or C₆) alkyl. In some embodiments, Z^{3a} is substituted or unsubstituted -(CH₂)ₘ(CH₂CH₂O)ₙ(CH₂)ₚ-, wherein m, n and p are integers independently selected from 1, 2, 3, 4, 5 and 6. In some embodiments, Z^{3a} is ethyl. In some embodiments, Z^{3a} is ethyl substituted by =O.

In some embodiments, Z^{1a}, Z^{2a}, Z^{4a} and Z^{5a} have a structure selected from Z'R^{20a}N(H)C(O)Z", Z'R^{20a}N(H)C(O)R^{21a}Z" and Z'R^{21a}Z" wherein Z' is a bond to the second scaffold moiety, Z" is a bond to one of the plurality of chelating moieties, R^{20a} is selected from substituted or unsubstituted alkyl and substituted or unsubstituted heteroalkyl. and R^{21a} is selected from substituted or unsubstituted alkyl and substituted or unsubstituted heteroalkyl. In some embodiments, R^{20a} is selected from substituted or unsubstituted (C₁, C₂, C₃, C₄, C₅ or C₆) alkyl and substituted or unsubstituted (C₁, C₂, C₃, C₄, C₅ or C₆) heteroalkyl. In some embodiments, R^{20a} is selected from substituted or unsubstituted ethyl. In some embodiments, R^{21a} is from substituted or unsubstituted - (CH₂)_{w}O- wherein w is selected from 1, 2, 3, 4, 5 and 6. In exemplary embodiments, w is 1 or 3.

In some embodiments, at least one of Z^{1a}, Z^{2a}, Z^{3a}, Z^{4a} and Z^{5a} is substituted by a linker.

Another particularly useful scaffold moiety for any chelator herein has the structure

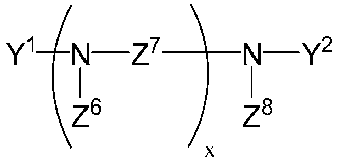

x is selected from 1, 2, 3 and 4. In exemplary embodiments, x is 1. In exemplary embodiments, x is 2. In exemplary embodiments, x is 3. In exemplary embodiments, x is 4.

Y¹ and Y² are each independently selected from H, substituted or unsubstituted alkyl and substituted or unsubstituted heteroalkyl. In exemplary embodiments, Y¹ and Y² are H.

Z⁷ is selected from substituted or unsubstituted alkyl and substituted or unsubstituted heteroalkyl. In exemplary embodiments, at least one Z⁷ is substituted by a linker. In some embodiments, each Z⁷ is independently substituted or unsubstituted (C₁, C₂, C₃, C₄, C₅ or C₆) alkyl. In exemplary embodiments, each Z⁷ is independently substituted or unsubstituted propyl or butyl. In some embodiments, each Z⁷ is independently substituted or unsubstituted heteroalkyl.

In exemplary embodiments, each Z⁷ is independently substituted or unsubstituted -(CH₂)ₘ(CH₂CH₂O)ₙ(CH₂)ₚ-, wherein m, n and p are integers independently selected from 1, 2, 3, 4, 5 and 6. In exemplary embodiments, each Z⁷ is substituted or unsubstituted -(CH₂)₂O(CH₂)₂-.

Z⁶ and Z⁸ are independently selected from -C(O)-, substituted or unsubstituted alkyl, and substituted or unsubstituted heteroalkyl; and each of Z⁶ and Z⁸ comprises a bond to one of the chelating moieties.

In exemplary embodiments, Z⁶ and Z⁸ are -C(O)-.

Another useful scaffold moiety has the structure: 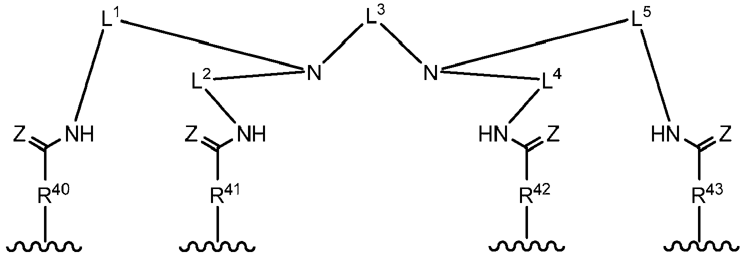 in which each Z is independently selected from O and S. In some embodiments, L³ comprises -(CH₂CH₂O)ₘR³¹- wherein m is an integer selected from 0, 1,2, 3, 4, 5, 6, 7, 8 and 9. In some embodiments, m is 0. In some embodiments, m is 1. In some embodiments, L³ is -CH₂CH₂OCH₂CH₂-. L¹, L², L⁴, L⁵ and R³¹ are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. In exemplary embodiments, L¹, L², L⁴, L⁵ are independently selected substituted or unsubstituted (C₁, C₂, C₃, C₄, C₅ or C₆) alkyl. In some embodiments, R³¹ is substituted or unsubstituted (C₁, C₂, C₃, C₄, C₅ or C₆) alkyl. In exemplary embodiments, L¹, L², L⁴, L⁵ are independently selected substituted or unsubstituted ethyl. In some embodiments, R³¹ is substituted or unsubstituted ethyl. In exemplary embodiments, L¹, L², L⁴, L⁵ are ethyl, one or more of which is substituted with a linker. In some embodiments, L¹ is substituted with a linker. In some embodiments, L² is substituted with a linker. In some embodiments, L³ is substituted with a linker. In some embodiments, L⁴ is substituted with a linker. In some embodiments, L⁵ is substituted with a linker. In some embodiments, L¹ is ethyl substituted with a linker. In some embodiments, L² is ethyl substituted with a linker. In some embodiments, L³ is ethyl substituted with a linker. In some embodiments, L⁴ is ethyl substituted with a linker. In some embodiments, L⁵ is ethyl substituted with a linker. In some embodiments, R⁴⁰, R⁴¹, R⁴² and R⁴³ are bonds. In some embodiments, R⁴⁰, R⁴¹, R⁴² and R⁴³ are -(CH₂)_{w}O-, wherein w is selected from 0, 1, 2, 3, 4, 5, 6, 7, 8, 9 and 10. In exemplary embodiments, w is 3.

Another useful scaffold has the structure 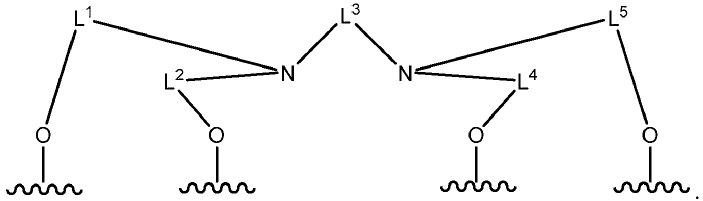 In some embodiments, L³ comprises -(CH₂CH₂O)ₘR³¹- wherein m is an integer selected from 0, 1,2, 3, 4, 5, 6, 7, 8 and 9. In some embodiments, m is 0. In some embodiments, m is 1. In some embodiments, L³ is -CH₂CH₂OCH₂CH₂-. In some embodiments, L³ is -C(O)C(O)-. L¹, L², L⁴, L⁵ and R³¹ are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. In exemplary embodiments, L¹, L², L⁴, L⁵ are independently selected substituted or unsubstituted (C₁, C₂, C₃, C₄, C₅ or C₆) alkyl. In some embodiments, R³¹ is substituted or unsubstituted (C₁, C₂, C₃, C₄, C₅ or C₆) alkyl. In exemplary embodiments, L¹, L², L⁴, L⁵ are independently selected substituted or unsubstituted ethyl. In exemplary embodiments, L¹, L², L⁴, L⁵ are independently selected substituted or unsubstituted propyl. In some embodiments, R³¹ is substituted or unsubstituted ethyl. In exemplary embodiments, L¹, L², L⁴, L⁵ are ethyl, one or more of which is substituted with a linker. In some embodiments, L¹ is substituted with a linker. In some embodiments, L² is substituted with a linker. In some embodiments, L³ is substituted with a linker. In some embodiments, L⁴ is substituted with a linker. In some embodiments, L⁵ is substituted with a linker. In some embodiments, L¹ is propyl substituted with a linker. In some embodiments, L² is propyl substituted with a linker. In some embodiments, L³ is propyl substituted with a linker. In some embodiments, L⁴ is propyl substituted with a linker. In some embodiments, L⁵ is propyl substituted with a linker.

In some embodiments, a scaffold is selected from: 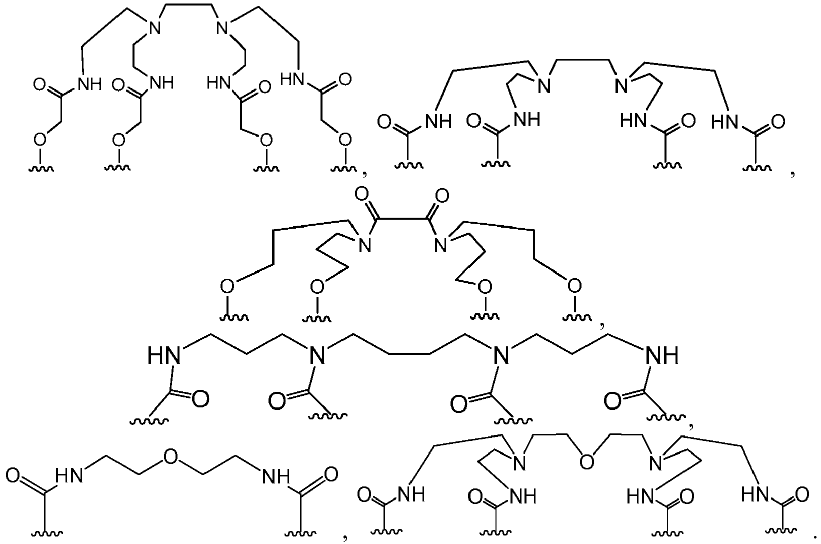 In any of these structures, one or more methyl, ethyl, propyl or butyl moieties can be substituted with one or more linkers. In some embodiments, two of these scaffold moieties, in which one or more methyl, ethyl, propyl or butyl moieties are optionally substituted with one or more linkers, are used to form a macrocycle.

### Linker

A "linker", "linking member", or "linking moiety" as used herein is a moiety that joins or potentially joins, covalently or noncovalently, a first moiety to a second moiety. In particular, a linker attaches or could potentially attach a chelator described herein to another molecule, such as a targeting moiety. In some embodiments, a linker attaches or could potentially attach a chelator described herein to a solid support. A linker comprising a reactive functional group that can be further reacted with a reactive functional group on a structure of interest in order to attach the structure of interest to the linker is referred to as a "functionalized linker". In exemplary embodiments, a linker is a functionalized linker. In exemplary embodiments, a chelator comprises one or more functionalized linkers. In some embodiments, a linker comprises a targeting moiety. In some embodiments, a linker to a targeting moiety comprises a bond to the targeting moiety.

A linker can be any useful structure for that joins a chelator to a reactive functional group or a targeting moiety, such as an antibody. Examples of a linker include O-order linkers (i.e., a bond), substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. Further exemplary linkers include substituted or unsubstituted (C₁, C₂, C₃, C₄, C₅, C₆, C₇, C₈, C₉ or C₁₀) alkyl, substituted or unsubstituted heteroalkyl, -C(O)NR'-, -C(O)O-, -C(O)S-, and -C(O)CR'R", wherein R' and R" are members independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, a linker includes at least one heteroatom. Exemplary linkers also include -C(O)NH-, -C(O), -NH-, -S-, -O-, and the like. In an exemplary embodiment, a linker is a heteroalkyl substituted with a reactive functional group.

### Reactive Functional Groups

In one embodiment, a linker comprises a reactive functional group (or a "reactive functional moiety", used synonymously). The reactive functional group can be further reacted to covalently attach the linker to another structure, such as a targeting moiety or a solid support, for example. Reactive functional groups and classes of reactions useful in practicing the present invention are generally those that are well known in the art of bioconjugate chemistry. Currently favored classes of reactions available with reactive functional groups of the invention are those which proceed under relatively mild conditions. These include, but are not limited to nucleophilic substitutions (e.g., reactions of amines and alcohols with acyl halides and activated esters), electrophilic substitutions (e.g., enamine reactions) and additions to carbon-carbon and carbon-heteroatom multiple bonds (e.g., Michael reactions and Diels-Alder reactions). These and other useful reactions are discussed, for example, in March, Advanced Organic Chemistry (3rd Ed., John Wiley & Sons, New York, 1985); Hermanson, Bioconjugate Techniques (Academic Press, San Diego, 1996); and Feeney et al., Modification of Proteins, Advances in Chemistry Series, Vol. 198 (American Chemical Society, Washington, D.C., 1982).

In some embodiments, a reactive functional group refers to a group selected from olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitro, nitriles, mercaptans, sulfides, disulfides, sulfoxides, sulfones, sulfonic acids, sulfinic acids, acetals, ketals, anhydrides, sulfates, sulfenic acids isonitriles, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines, azides, azo compounds, azoxy compounds, and nitroso compounds. Reactive functional groups also include those used to prepare bioconjugates, e.g., N-hydroxysuccinimide esters, maleimides and the like. Methods to prepare each of these functional groups are well known in the art and their application or modification for a particular purpose is within the ability of one of skill in the art (*see,* for example, Sandler and Karo, eds., Organic Functional Group Preparations, (Academic Press, San Diego, 1989)).

A reactive functional group can be chosen according to a selected reaction partner. As an example, an activated ester, such as an NHS ester will be useful to label a protein *via* lysine residues. Sulfhydryl reactive groups, such as maleimides can be used to label proteins *via* amino acid residues carrying an SH-group (e.g., cystein). Antibodies may be labeled by first oxidizing their carbohydrate moieties (e.g., with periodate) and reacting resulting aldehyde groups with a hydrazine containing ligand.

The reactive functional groups can be chosen such that they do not participate in, or interfere with, the reactions necessary to assemble the reactive ligand. Alternatively, a reactive functional group can be protected from participating in the reaction by means of a protecting group. Those of skill in the art understand how to protect a particular functional group so that it does not interfere with a chosen set of reaction conditions. For examples of useful protecting groups, see, for example, Greene et al., PROTECTIVE GROUPS IN ORGANIC SYNTHESIS, John Wiley & Sons, New York, 1991.

### Amines and Amino-Reactive Groups

In one embodiment, a reactive functional group is selected from an amine, (such as a primary or secondary amine), hydrazine, hydrazide and sulfonylhydrazide. Amines can, for example, be acylated, alkylated or oxidized. Useful non-limiting examples of amino-reactive groups include N-hydroxysuccinimide (NHS) esters, sulfur-NHS esters, imidoesters, isocyanates, isothiocyanates, acylhalides, arylazides, p-nitrophenyl esters, aldehydes, sulfonyl chlorides, thiazolides and carboxyl groups.

NHS esters and sulfur-NHS esters react preferentially with a primary (including aromatic) amino groups of a reaction partner. The imidazole groups of histidines are known to compete with primary amines for reaction, but the reaction products are unstable and readily hydrolyzed. The reaction involves the nucleophilic attack of an amine on the acid carboxyl of an NHS ester to form an amide, releasing the N-hydroxysuccinimide.

Imidoesters are the most specific acylating reagents for reaction with amine groups of a molecule such as a protein. At a pH between 7 and 10, imidoesters react only with primary amines. Primary amines attack imidates nucleophilically to produce an intermediate that breaks down to amidine at high pH or to a new imidate at low pH. The new imidate can react with another primary amine, thus crosslinking two amino groups, a case of a putatively monofunctional imidate reacting bifunctionally. The principal product of reaction with primary amines is an amidine that is a stronger base than the original amine. The positive charge of the original amino group is therefore retained. As a result, imidoesters do not affect the overall charge of the conjugate.

Isocyanates (and isothiocyanates) react with the primary amines of the conjugate components to form stable bonds. Their reactions with sulfhydryl, imidazole, and tyrosyl groups give relatively unstable products.

Acylazides are also used as amino-specific reagents in which nucleophilic amines of the reaction partner attack acidic carboxyl groups under slightly alkaline conditions, e.g. pH 8.5.

Arylhalides such as 1,5-difluoro-2,4-dinitrobenzene react preferentially with the amino groups and tyrosine phenolic groups of the conjugate components, but also with its sulfhydryl and imidazole groups.

p-Nitrophenyl esters of carboxylic acids are also useful amino-reactive groups. Although the reagent specificity is not very high, α- and ε-amino groups appear to react most rapidly.

Aldehydes react with primary amines of the conjugate components (*e.g*., ε-amino group of lysine residues). Although unstable, Schiff bases are formed upon reaction of the protein amino groups with the aldehyde. Schiff bases, however, are stable, when conjugated to another double bond. The resonant interaction of both double bonds prevents hydrolysis of the Schiff linkage. Furthermore, amines at high local concentrations can attack the ethylenic double bond to form a stable Michael addition product. Alternatively, a stable bond may be formed by reductive amination.

Aromatic sulfonyl chlorides react with a variety of sites of the conjugate components, but reaction with the amino groups is the most important, resulting in a stable sulfonamide linkage.

Free carboxyl groups react with carbodiimides, soluble in both water and organic solvents, forming pseudoureas that can then couple to available amines yielding an amide linkage. Yamada et al., Biochemistry, 1981, 20: 4836-4842, e.g., teach how to modify a protein with carbodiimides.

### Sulfhydryl and Sulfhydryl-Reactive Groups

In another embodiment, a reactive functional group is selected from a sulfhydryl group (which can be converted to disulfides) and sulfhydryl-reactive group. Useful non-limiting examples of sulfhydryl-reactive groups include maleimides, alkyl halides, acyl halides (including bromoacetamide or chloroacetamide), pyridyl disulfides, and thiophthalimides.

Maleimides react preferentially with the sulfhydryl group of the conjugate components to form stable thioether bonds. They also react at a much slower rate with primary amino groups and the imidazole groups of histidines. However, at pH 7 the maleimide group can be considered a sulfhydryl-specific group, since at this pH the reaction rate of simple thiols is 1000-fold greater than that of the corresponding amine.

Alkyl halides react with sulfhydryl groups, sulfides, imidazoles, and amino groups. At neutral to slightly alkaline pH, however, alkyl halides react primarily with sulfhydryl groups to form stable thioether bonds. At higher pH, reaction with amino groups is favored.

Pyridyl disulfides react with free sulfhydryl groups via disulfide exchange to give mixed disulfides. As a result, pyridyl disulfides are relatively specific sulfhydryl-reactive groups.

Thiophthalimides react with free sulfhydryl groups to also form disulfides.

### Other Reactive Functional Groups

Other exemplary reactive functional groups include:
(i) carboxyl groups and various derivatives thereof including, but not limited to, N-hydroxybenztriazole esters, acid halides, acyl imidazoles, thioesters, p-nitrophenyl esters, alkyl, alkenyl, alkynyl and aromatic esters;
(ii) hydroxyl groups, which can be converted to esters, ethers, aldehydes, etc.;
(iii) haloalkyl groups, wherein the halide can be displaced with a nucleophilic group such as, for example, an amine, a carboxylate anion, thiol anion, carbanion, or an alkoxide ion, thereby resulting in the covalent attachment of a new group at the site of the halogen atom;
(iv) dienophile groups, which are capable of participating in Diels-Alder reactions such as, for example, maleimido groups;
(v) aldehyde or ketone groups, such that subsequent derivatization is possible via formation of carbonyl derivatives such as, for example, imines, hydrazones, semicarbazones or oximes, or via such mechanisms as Grignard addition or alkyllithium addition;
(vi) alkenes, which can undergo, for example, cycloadditions, acylation, Michael addition, etc;
(vii) epoxides, which can react with, for example, amines and hydroxyl groups;
(ix) phosphoramidites and other standard functional groups useful in nucleic acid synthesis and
(x) any other functional group useful to form a covalent bond between the functionalized ligand and a molecular entity or a surface.

### Functional Groups with Non-specific Reactivities

In addition to the use of site-specific reactive moieties, the present invention contemplates the use of non-specific reactive groups to link a chelator to a targeting moiety. Non-specific groups include photoactivatable groups, for example.

Photoactivatable groups are ideally inert in the dark and are converted to reactive species in the presence of light. In one embodiment, photoactivatable groups are selected from precursors of nitrenes generated upon heating or photolysis of azides. Electron-deficient nitrenes are extremely reactive and can react with a variety of chemical bonds including N-H, O-H, C-H, and C=C. Although three types of azides (aryl, alkyl, and acyl derivatives) may be employed, arylazides are presently preferrred. The reactivity of arylazides upon photolysis is better with N-H and O-H than C-H bonds. Electron-deficient arylnitrenes rapidly ring-expand to form dehydroazepines, which tend to react with nucleophiles, rather than form C-H insertion products. The reactivity of arylazides can be increased by the presence of electron-withdrawing substituents such as nitro or hydroxyl groups in the ring. Such substituents push the absorption maximum of arylazides to longer wavelength. Unsubstituted arylazides have an absorption maximum in the range of 260-280 nm, while hydroxy and nitroarylazides absorb significant light beyond 305 nm. Therefore, hydroxy and nitroarylazides are most preferable since they allow to employ less harmful photolysis conditions for the affinity component than unsubstituted arylazides.

In another preferred embodiment, photoactivatable groups are selected from fluorinated arylazides. The photolysis products of fluorinated arylazides are arylnitrenes, all of which undergo the characteristic reactions of this group, including C-H bond insertion, with high efficiency (Keana et al., J. Org. Chem. 55: 3640-3647, 1990).

In another embodiment, photoactivatable groups are selected from benzophenone residues. Benzophenone reagents generally give higher crosslinking yields than arylazide reagents.

In another embodiment, photoactivatable groups are selected from diazo compounds, which form an electron-deficient carbene upon photolysis. These carbenes undergo a variety of reactions including insertion into C-H bonds, addition to double bonds (including aromatic systems), hydrogen attraction and coordination to nucleophilic centers to give carbon ions.

In still another embodiment, photoactivatable groups are selected from diazopyruvates. For example, the p-nitrophenyl ester of p-nitrophenyl diazopyruvate reacts with aliphatic amines to give diazopyruvic acid amides that undergo ultraviolet photolysis to form aldehydes. The photolyzed diazopyruvate-modified affinity component will react like formaldehyde or glutaraldehyde forming intraprotein crosslinks.

In exemplary embodiments, a linker joins a chelator to a targeting moiety. That is, in exemplary embodiments, a linker comprises a targeting moiety. In some embodiments, a chelator comprises a linker to a targeting moiety. Any linker described herein may be a linker comprising a reactive functional group that could react with a reactive functional group on a targeting moiety to join the linker to the targeting moiety. Any linker described herein may be a linker comprising a bond to a targeting moiety. The term "targeting moiety" refers to a moiety serves to target or direct the molecule to which it is attached (e.g. a chelator or a chelator complexed to a radionuclide) to a particular location or molecule. Thus, for example, a targeting moiety may be used to target a molecule to a specific target protein or enzyme, or to a particular cellular location, to a particular cell type or to a diseased tissue. As will be appreciated by those in the art, the localization of proteins within a cell is a simple method for increasing effective concentration. For example, shuttling an imaging agent and/or therapeutic into the nucleus confines them to a smaller space thereby increasing concentration. Finally, the physiological target may simply be localized to a specific compartment, and the agents must be localized appropriately.

The targeting moiety can be a small molecule (e.g., MW < 500D), which includes both non-peptides and peptides. Examples of a targeting moiety also include peptides, polypeptides (including proteins, and in particular antibodies, which includes antibody fragments), nucleic acids, oligonucleotides, carbohydrates, lipids, hormones (including proteinaceous and steroid hormones), growth factors, lectins, receptors, receptor ligands, cofactors and the like. Targets of a targeting moiety can include a complementary nucleic acid, a receptor, an antibody, an antigen or a lectin, for example.

In exemplary embodiments, a targeting moiety can bind to a target with high binding affinity. In other words, a targeting moiety with high binding affinity to a target has a high specificity for or specifically binds to the target. In some embodiments, a high binding affinity is given by a dissociation constant K_{d} of about 10⁻⁷ M or less. In exemplary embodiments, a high binding affinity is given by a dissociation constant K_{d} of about 10⁻⁸ M or less, about 10⁻⁹ M or less, about 10⁻¹⁰ M or less, about 10⁻¹¹ M or less, about 10⁻¹² M or less, about 10⁻¹³ M or less, about 10⁻¹⁴ M or less or about 10⁻¹⁵ M or less. A compound may have a high binding affinity for a target if the compound comprises a portion, such as a targeting moiety, that has a high binding affinity for the target.

In exemplary embodiments, a targeting moiety is an antibody. An "antibody" refers to a protein comprising one or more polypeptides substantially encoded by all or part of the recognized immunoglobulin genes. The recognized immunoglobulin genes, for example in humans, include the kappa (κ), lambda (λ) and heavy chain genetic loci, which together compose the myriad variable region genes, and the constant region genes mu (µ), delta (δ), gamma (γ), epsilon (ε) and alpha (α), which encode the IgM, IgD, IgG, IgE, and IgA isotypes respectively. Antibody herein is meant to include full length antibodies and antibody fragments, and may refer to a natural antibody from any organism, an engineered antibody or an antibody generated recombinantly for experimental, therapeutic or other purposes as further defined below. Antibody fragments include Fab, Fab', F(ab')₂, Fv, scFv or other antigen-binding subsequences of antibodies and can include those produced by the modification of whole antibodies or those synthesized de novo using recombinant DNA technologies. The term "antibody" refers to both monoclonal and polyclonal antibodies. Antibodies can be antagonists, agonists, neutralizing, inhibitory or stimulatory.

While a targeting moiety may be appended to a chelator in order to localize the compound to a specific region in an animal, certain chelators have a natural affinity for cells, tissue, organs or some other part of the animal. For example, a chelator disclosed herein might have a natural or intrinsic affinity for bone. Thus, in some embodiments, a chelator, such as an open chelator or a macrocycle, does not comprise a targeting moiety or a linker to a targeting moiety. A chelator lacking a targeting moiety can be used in any method that does not require specific targeting.

In some embodiments, a chelator comprises a linker to a solid support. That is, any linker described herein may be a linker comprising a reactive functional group that could react with a reactive functional group on a solid support to join the linker to the solid support. Any linker described herein may be a linker comprising a bond to a solid support. A "solid support" is any material that can be modified to contain discrete individual sites suitable for the attachment or association of a chelator. Suitable substrates include biodegradable beads, non-biodegradable beads, silica beads, magnetic beads, latex beads, glass beads, quartz beads, metal beads, gold beads, mica beads, plastic beads, ceramic beads, or combinations thereof. Of particular use are biocompatible polymers, including biodegradable polymers that are slowly removed from the system by enzymatic degradation. Example biodegradable materials include starch, cross-linked starch, poly(ethylene glycol), polyvinylpyrrolidine, polylactides (PLA), polyglycolides (PGA), poly(lactide-co-glycolides) (PLGA), polyanhydrides, polyorthoesters, poly(DTH iminocarbonate), poly(bisphenol A iminocarbonate), polycyanoacrylate, polyphosphazene, mixtures thereof and combinations thereof. Other suitable substances for forming the particles exist and can be used. In some embodiments, a solid support is a bead comprising a cross-linked starch, for example, cross-linked potato starch. Beads made from starch are completely biodegradable in the body, typically by serum amylase, a naturally occurring enzyme found in the body. In these embodiments, the chelator optionally further comprises a targeting moiety or a linker to a targeting moeity. In cases where a chelator that is attached to a solid support does not comprise a targeting moiety, the chealtor can be localized directly by the practitioner, for example, by direct surgical implantation.

In some embodiments, a linker has the structure -L¹¹-X, wherein L¹¹ is selected from a bond, acyl, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; and X is a reactive functional group or a targeting moiety.

In some embodiments, L¹¹ is selected from substituted or unsubstituted alkyl and substituted or unsubstituted heteroalkyl. In some embodiments, L¹¹ is heteroalkyl. In some embodiments, L¹¹ is (C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉ or C₂₀) alkyl in which 1, 2 or 3 atoms are replaced with a heteroatom, such as nitrogen or oxygen.

In some embodiments, X is selected from -NH₂ and -CO(O)H.

In some embodiments, -L¹¹-X is selected from

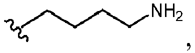

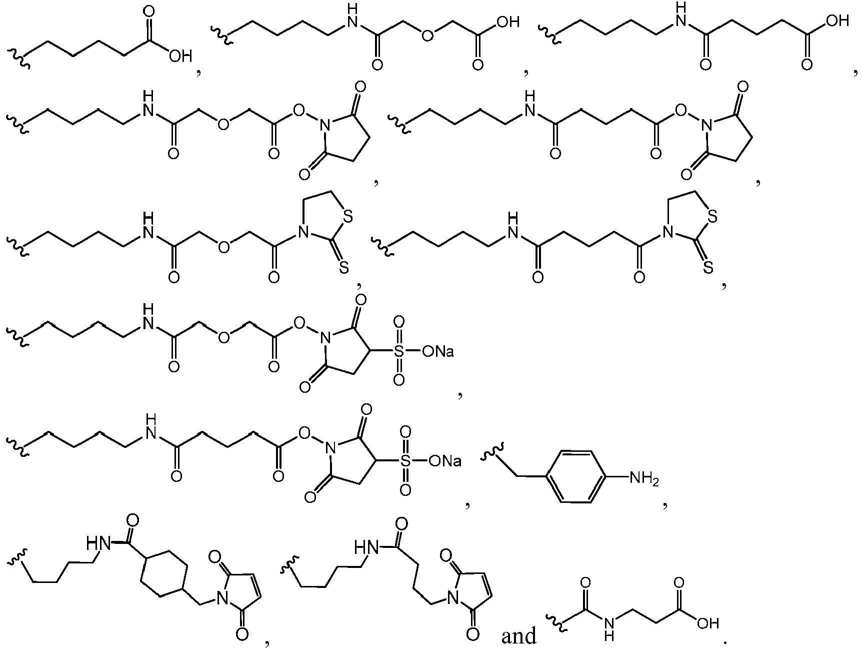

In exemplary embodiments, X is a targeting moiety.

In exemplary embodiments, a linker is a linker to a targeting moiety. In some embodiments, the targeting moiety is selected from a polypeptide, a nucleic acid, a lipid, a polysaccharide, a small molecule, a cofactor and a hormone. In exemplary embodiments, the targeting moiety is an antibody or antibody fragment.

In some embodiments, a linker includes an aliphatic carbon chain or a polyethyleneglycol (PEG) chain. Thus, a linker can comprise a structure selected from: 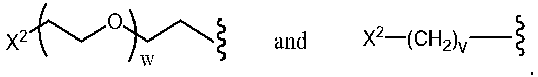 The integer v is selected from 1 to 20, and w is an integer from 1 to 1,000 or 1 to 500 or 1 to 100 or 1 to 50 or 1 to 10.

Exemplary X² groups include OH, alkoxy, and one of the following structures: 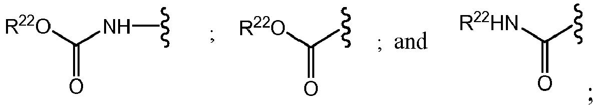 wherein R²² is a member selected from H, substituted or unsubstituted alkyl, substituted or unsubstituted heteroalkyl, substituted or unsubstituted aryl, substituted or unsubstituted heteroaryl and substituted or unsubstituted heterocycloalkyl. The integer v is selected from 1 to 20, and w is an integer from 1 to 1,000 or 1 to 500 or 1 to 100 or 1 to 50 or 1 to 10.

In some embodiments, a linker has the structure: 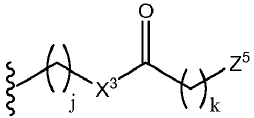 wherein Z⁵ is selected from H, OR²³, SR²³, NHR²³, OCOR²¹, OC(O)NHR²⁴, NHC(O)OR²³, OS(O)₂OR²³, and C(O)R²⁴. R²³ is selected from H, substituted or unsubstituted alkyl, and substituted or unsubstituted heteroalkyl. R²⁴ is selected from H, OR²⁵, NR²⁵NH₂, SH, C(O)R²⁵, NR²⁵H, substituted or unsubstituted alkyl and substituted or unsubstituted heteroalkyl. R²⁵ is selected from H, substituted or unsubstituted alkyl and substituted or unsubstituted alkyl. X³ is selected from O, S and NR²⁶, wherein R²⁶ is a member selected from H, substituted or unsubstituted alkyl and substituted or unsubstituted heteroalkyl. The integers j and k are members independently selected from 1, 2, 3, 4, 5, 6, 7, 8, 9, 10, 11, 12, 13, 14, 15, 16, 17, 18, 19 and 20. In some embodiments, the integers j and k are members independently selected from 1, 2, 3, 4, 5,6.

In a linker with multiple reactive functional groups, a particular functional group can be chosen such that it does not participate in, or interfere with, the reaction controlling the attachment of the functionalized spacer component to another ligand component. Alternatively, the reactive functional group can be protected from participating in the reaction by the presence of a protecting group. Those of skill in the art understand how to protect a particular functional group from interfering with a chosen set of reaction conditions. For examples of useful protecting groups, *See* Greene et al., PROTECTIVE GROUPS IN ORGANIC SYNTHESIS, John Wiley & Sons, New York, 1991.

### Radionuclides

The chelating moieties disclosed herein can be used to bind metal ions, in particular, a radionuclide. The term "radionuclide" or "radioisotope" refers to a radioactive isotope or element with an unstable nucleus that tends to undergo radioactive decay. Numerous decay modes are known in the art and include alpha decay, proton emission, neutron emission, double proton emission, spontaneous fission, cluster decay, β⁻ decay, positron emission (β⁺ decay), electron capture, bound state beta decay, double beta decay, double electron capture, electron capture with positron emission, double positron emission, isomeric transition and internal conversion.

Exemplary radionuclides include alpha-emitters, which emit alpha particles during decay. In some embodiments, a radionuclide is an emitter of a gamma ray or a particle selected from an alpha particle, an electron and a positron.

In some embodiments, the radionuclide is an actinide. In some embodiments, the radionuclide is a lanthanide. In some embodiments, the radionuclide is a 3⁺ ion. In some embodiments, the radionuclide is a 4⁺ ion. In some embodiments the radionuclide is a 2⁺ ion.

Of particular use in the complexes provided herein are radionuclides selected from isotopes of U, Pu, Fe, Cu, Ce, Nd, Eu, Sm, Gd, Tb, Dy, Ho, Er, Yb, Lu, Y, Th, Zr, In, Ga, Bi, Ra and Ac. In some embodiments, one or more of these radionuclides are excluded. In some embodiments, a radionuclide is selected form radium-223, thorium-227, bismuth-213, Lutetium-177, and actinium-225. Other useful radioisotopes include bismuth-212, iodine-123, copper-64, iridium-192, osmium-194, rhodium-105, samarium-153, and yttrium-88, yttrium-90, and yttrium-91. In exemplary embodiments, the radionuclide is thorium, particularly selected from thorium-227 and thorium-232. In some embodiments, thorium-226 is excluded. In some embodiments, U is excluded. In some embodiments, uranium-230 is excluded. That is, in some embodiments, a radionuclide is not U, or a radionuclide is not uranium-230 or a radionuclide is not thorium-226.

²³²Th exists in nature as an α-emitter with a half life of 1.4 x 10¹⁰ yr. In aqueous solution, Th(IV) is the only oxidation state. Thorium(IV) ion is bigger than Pu(IV) and usually forms complexes with 9 or higher coordination number. For example, the crystal structure of both Th(IV) complexes of simple bidentate 1,2-HOPO and Me-3,2-HOPO have been determined as nine coordinated species.

Similar to other actinide ions, thorium(IV) prefers forming complexes with oxygen, especially negative oxygen donor ligands. Thorium(IV) also prefers octadentate or higher multidentate ligands:

| Ligand | **Acac** | **NTA** | **HEDTA*** | **EDTA**** | **DTPA** | **TTHA** |
|---|---|---|---|---|---|---|
| Ligand Type | Bi-dentate | Tetra- | Hexa- | Hexa- | Octa- | Deca- |
| **LogK₁** | **7.85** | **16.9** | **18.5** | **25.3** | **30.34** | **31.9** |

| | | | | | | |
|---|---|---|---|---|---|---|
| *with one alcoholic oxygen and three carboxyl groups; **with four carboxyl groups. | | | | | | |

Other radionuclides with diagnostic and therapeutic value that can be used with the compounds disclosed herein can be found, for example, in U.S. Patent Nos. 5,482,698 and 5,601,800; and Boswell and Brechbiel, Nuclear Medicine and Biology, 2007 October, 34(7): 757-778 and the manuscript thereof made available in PMC 2008 October 1.

### Uses

The chelators and complexes disclosed herein can be used in a wide variety of therapeutic and diagnostic settings.

In one aspect, the invention provides a method of treating a disease in an animal comprising administering a complex disclosed herein to the animal, whereby the disease is ameliorated or eliminated.

In one aspect, the invention provides a method of diagnosing a disease in an animal comprising (a) administering a complex disclosed herein to the animal and (b) detecting the presence or absence of a signal emitted by the complex. In some embodiments, the detecting step comprises obtaining an image based on the signal.

In some embodiments, the disease is cancer.

In some embodiments, the complex comprises a linker to a targeting moiety and the method further comprises localizing the complex to a targeting site in the animal by binding the targeting moiety to the targeting site.

The compounds disclosed herein are particularly well suited for the preparation of stable, pre-labeled antibodies for use in the diagnosis and treatment of cancer and other diseases. For example, antibodies expressing affinity for specific tumors or tumor-associated antigens are labeled with a diagnostic radionuclide-complexed chelate, and the labeled antibodies can be further stabilized through lyophilization. Where a chelate is used, it generally is covalently attached to the antibody. The antibodies used can be polyclonal or monoclonal, and the radionuclide-labeled antibodies can be prepared according to methods known in the art. The method of preparation will depend upon the type of radionuclide and antibody used. A stable, lyophilized, radiolabeled antibody can be reconstituted with suitable diluent at the time of intended use, thus greatly simplifying the on site preparation process. The methods of the invention can be applied to stabilize many types of pre-labeled antibodies, including, but not limited to, polyclonal and monoclonal antibodies to tumors associated with melanoma, colon cancer, breast cancer, prostate cancer, etc. Such antibodies are known in the art and are readily available.

### Synthesis

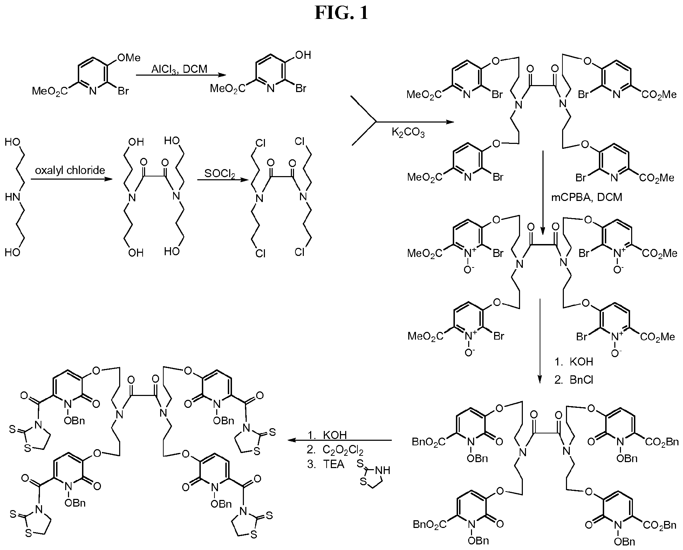

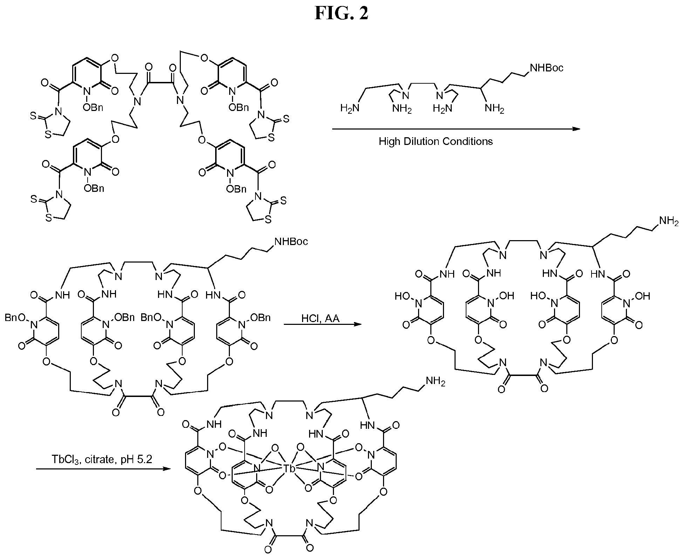

Methods for synthesizing the chelating moieties disclosed herein are known in the art. See, for example, WO/2008/008797; US Patent 6,846,915; US Patent 7,404,912; and US Patent 5,010,191. For forming a chelator, figures 1-4 show general schematics in which a scaffold moiety containing reactive functional groups such as halogen or amine can be reacted with a chelating moiety having a reactive function group that will result in covalent binding of the moieties.

Any scaffold moiety can be derivatized with at least one linker, such as a functionalized linker. Thus, in one exemplary embodiment, a linker, such as a functionalized linker, can be attached to the scaffold moiety. In another exemplary embodiment, a linker, such as a functionalized linker, is attached to a chelating moiety. A functionalized linker can reacted to form a bond with a targeting moiety. The linker can also be attached to any other linker within a compound.

Scaffold moieties that include a linker can be prepared by the following exemplary methods.

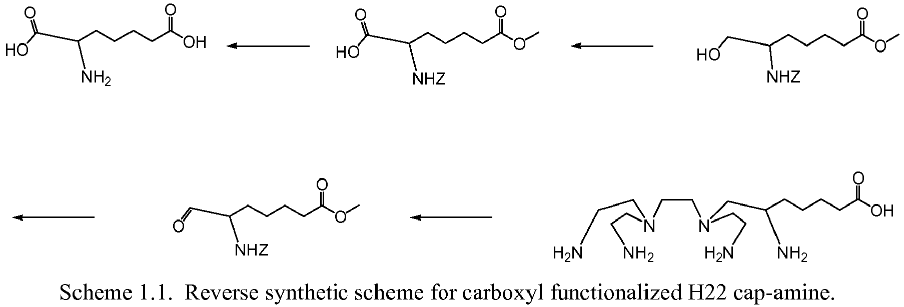

Other functionalize scaffolds include those in which the chiral carbon is placed on the central ethylene bridge of H22-amine. An exemplary route to such a scaffold initiates with 2,3-Diaminopropionic acid, as its carboxyl group is connected directly to the amine backbone to give a very rigid geometry, extended carboxyl chain is needed to provide flexibility for eventual protein conjugating. A synthetic scheme to the scaffold is shown in scheme 1.2.

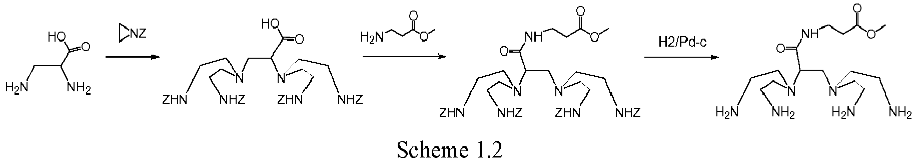

Variations on this synthesis include the use of a nitrophenylalanine or a BOC-amino group, which are optionally converted to carboxyl groups. Synthetic routes to these scaffolds are shown in Schemes 1.3 and 1.4.

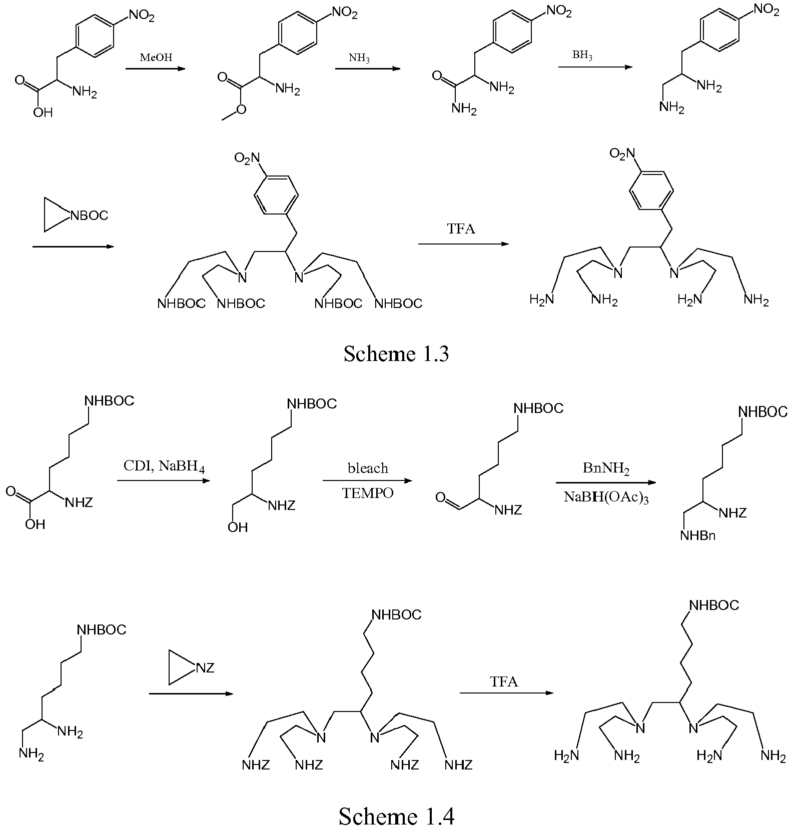

One concern with HOPO chelating moieties is that it might be difficult to couple these to a targeting moiety, such as an antibody, without protection in some form or another. One approach for HOPO chelating moiety protection/deprotection is to use a metal complex in the coupling reaction, then remove the metal from the metal complex-antibody conjugate after coupling to make room for the radionuclide (transmetalation). Another approach is to use ortho-nitrobenzyl in place of the benzyl protective group in the HOPO chelating moiety synthesis, and photodeprotect this after coupling the potential chelating moiety to the antibody.

Additional guidance for deprotecting, activating and attaching one or more chelating moieties to one or more scaffolds can be found, for example in US Patents 5,624,901; 6,406,297; 6,515,113 and 6,846,915; US Patent Application Publications 2008/0213780; 2008/0213917 and 2010/0015725; and PCT/US2010/046517.

Exemplary open chelators and macrocycles, any of which can be derivatized with a linker (e.g., a functionalized liker or a linker comprising a targeting moiety) are disclosed throughout the application.

### EXAMPLES

### Example 1

### Synthesis of a 1,2 HOPO trimacrocyclic chelator

The compounds and complexes of the invention are synthesized by an appropriate combination of generally well-known synthetic methods. Techniques useful in synthesizing the compounds of the invention are both readily apparent and accessible to those of skill in the relevant art. The discussion below is offered to illustrate certain of the diverse methods available for use in assembling the compounds of the invention, it is not intended to limit the scope of reactions or reaction sequences that are useful in preparing the compounds of the present invention.

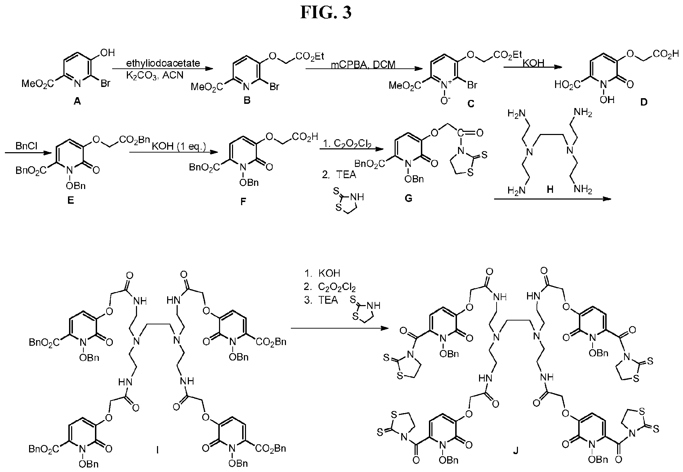

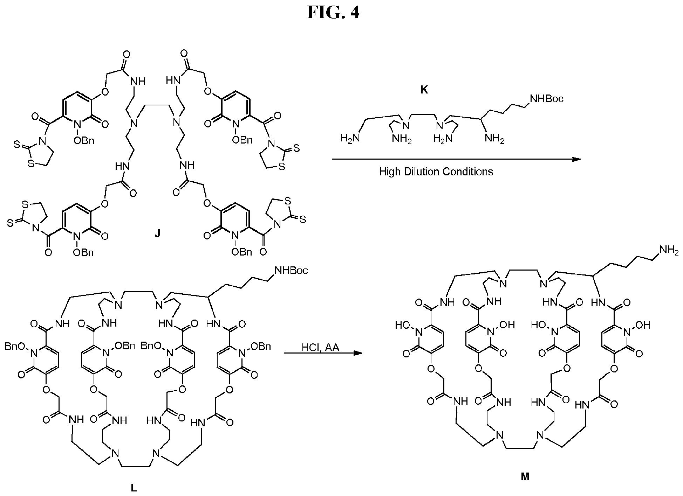

Figures 3 and 4 show one possible multistep synthetic route for synthesizing a 1,2-HOPO macrocycle.

### Methyl 2-bromo-3-ethylacetoxy-6-pyridinecarboxylate (B)

To a mixture of 1 molar equivalent of methyl 2-bromo-3-hydroxy-6-pyridine carboxylate **A** (prepared as described in Kelly, T.R.; Lang, F. J. Org. Chem. 1996, 61, 4623-4633), potassium carbonate (3 molar equivalent), and anhydrous acetonitrile is added ethyl 2-iodoacetate (Aldrich Chemical Company, 1.5 molar equivalents). The resulting suspension is heated at reflux for several hours until the starting material is consumed as judged by thin layer chromatography. Solvent is removed by rotary evaporation, and the residue dissolved in dichloromethane and water. The solvents are separated, and the dichloromethane fraction concentrated by rotary evaporation and purified by silica gel column chromatography to yield compound **B.**

### Methyl 2-bromo-3-ethylacetoxy-6-pyridine-N-oxidecarboxylate (C)

Methyl 2-bromo-3-ethylacetoxy-6-pyridinecarboxylate **B** is dissolved in dichloromethane and 3-chloroperoxybenzoic acid (3 molar equivalents) is added to this solution with stirring at ambient temperature. After the reaction finished, as judged by thin layer chromatography, the reaction mixture is concentrated by rotary evaporation and washed with water. The organic phase is concentrated further and the product **C** is purified using silica gel column chromatography.

### 1-Hydroxy-3-acetoxy-6-carboxy-2(1H)pyridinone (D)

Methyl 2-bromo-3-ethylacetoxy-6-pyridine-N-oxidecarboxylate **C** is dissolved in a solution of tetrahydrofuran and 10% aqueous KOH. The resulting solution is heated at 80 °C and the hydrolysis reaction monitored by thin layer chromatography. Upon reaction completion, tetrahydrofuran is removed under reduced pressure. The resulting mixture is cooled in an ice bath and treated with concentrated HC1 until the pH of the solution reaches 2. The resulting solid is isolated by filtration, washed with dilute HC1 followed by cold water, and then dried in vacuo to yield compound **D.**

### 1-Benzyloxy-3-acetoxy-6-carboxy-2(1H)-pyridinone dibenzyl ester (E)

1-Hydroxy-3-acetoxy-6-carboxy-2(1H)pyridinone **(D)** and anhydrous potassium carbonate (3 molar equivalents) are mixed with benzyl chloride (3 molar equivalents) in DMF. The mixture is heated at 80 °C for 1 day, filtered, and the filtrate evaporated to dryness. The residue is partitioned between 4 M aqueous potassium carbonate and dichloromethane. The aqueous phase is extracted with dichloromethane and the combined organic phases are concentrated by rotary evaporation. Compound **D** is purified by silica gel column chromatography.

### 1-Benzyloxy-3-acetoxy-6-carboxy-2(1H)-pyridinone benzyl ester (F)

1-Benzyloxy-3-acetoxy-6-carboxy-2(1H)-pyridinone dibenzyl ester **(E)** is dissolved in a solution of tetrahydrofuran and water. KOH (1 molar equivalent) is added, the solution is stirred at ambient temperature, and the hydrolysis reaction monitored by thin layer chromatography. Upon reaction completion, tetrahydrofuran is removed under reduced pressure. The resulting mixture is cooled in an ice bath and treated with concentrated HCl until the pH of the solution reaches 2. The resulting solid is isolated by filtration, washed with dilute HCl followed by cold water, and then dried in vacuo to yield compound **F.**

### 1-Benzyloxy-3-[2-mercaptothiazole]acetoxy-6-carboxy-2(1H)-pyridinone benzyl ester (G)

1-Benzyloxy-3-acetoxy-6-carboxy-2(1H)-pyridinone benzyl ester **(F)** is dissolved in anhydrous dioxane. Thionyl chloride (1.2 molar equivalents) and a drop of dimethylformamide are added, the solution is stirred at reflux, and the reaction monitored by thin layer chromatography. Upon reaction completion, all solvents are removed under reduced pressure and the residue is dried in vacuo. The resulting residue is dissolved in dry tetrahydrofuran and added dropwise to a solution of 2-mercaptothiazoline (1.1 molar equivalents) and triethylamine (1.1 molar equivalents) in tetrahydrofuran that is cooled in an ice bath. Upon reaction completion, all solvents are removed under reduced pressure and the residue is purified by silica gel column chromatography to yield compound **G.**

### Tetrakis(2-[1-benzyloxy-3-acetoxyamid-yl-6-carboxy-2(1H)-pyridinone benzyl ester] ethyl)ethylene diamine (I)

1-Benzyloxy-3-[2-mercaptothiazole]acetoxy-6-carboxy-2(1H)-pyridinone benzyl ester **(G)** is dissolved in anhydrous dichloromethane. A solution of tetrakis(2-aminoethyl)ethylene diamine (**H,** 0.25 molar equivalent, Wagnon, B.K.; Jackels, S.C. Inorg. Chem. 1989, 28, 1923-1927) and triethylamine (4 molar equivalents) is added, the solution is stirred at ambient temperature, and the condensation reaction monitored by thin layer chromatography. Upon reaction completion, all solvents are removed under reduced pressure and the residue is dried in vacuo. The resulting residue is purified by silica gel column chromatography to yield compound **I**.

### Tetrakis(2-[1-benzyloxy-3-acetoxyamid-yl-6-{2-mercaptothiazole}carboxy-2(1H)-pyridinone]ethyl)ethylene diamine (J)

Tetrakis(2-[1-benzyloxy-3-acetoxyamid-yl-6-carboxy-2(1H)-pyridinone benzyl ester] ethyl)ethylene diamine **(I)** is dissolved in a solution of tetrahydrofuran and water. KOH (4 molar equivalents) is added, the solution is stirred at ambient temperature, and the hydrolysis reaction monitored by thin layer chromatography. Upon reaction completion, tetrahydrofuran is removed under reduced pressure. The resulting mixture is cooled in an ice bath and treated with concentrated HC1 until the pH of the solution reaches 2. The resulting solid is isolated by filtration, washed with dilute HC1 followed by cold water, and then dried in vacuo. The resulting residue is dissolved in anhydrous dioxane. Thionyl chloride (4.8 molar equivalents) and a drop of dimethylformamide are added, the solution is stirred at reflux, and the reaction monitored by thin layer chromatography. Upon reaction completion, all solvents are removed under reduced pressure and the residue is dried in vacuo. The resulting residue is dissolved in dry tetrahydrofuran and added dropwise to a solution of 2-mercaptothiazoline (4.4 molar equivalents) and triethylamine (4.4 molar equivalents) in tetrahydrofuran that is cooled in an ice bath. Upon reaction completion, all solvents are removed under reduced pressure and the residue is purified by silica gel column chromatography to yield compound **J.**

### Trimacrocyclic compound (L)

Tetrakis(2-[1-benzyloxy-3-acetoxyamid-yl-6-{2-mercaptothiazole}carboxy-2(1H)-pyridinone]ethyl)ethylene diamine **(J)** is dissolved in 950 mL of chloroform in a round bottom flask. The free base form of [5-amino-6-((2-amino-ethyl)-{2-[bis-(2-amino-ethyl)-amino]-ethyl}-amino)-hexyl]-carbamic acid tert-butyl ester **(K)** (1 molar equivalent, WO2008/063721) is dissolved in triethylamine (12 molar equivalents), chloroform, and isopropyl alcohol in a separate round bottom flask. The solutions **of J** and **K** (c.a. 3-4 mM) are added simultaneously to a three-neck round flask containing additional dichloromethane (4 times the total volume of the solutions **of J** and **K**) and triethylamine (3 molar equivalents) over the course of 8-10 days. It is necessary to maintain high dilution conditions in order to minimize polymeric by-products. Upon reaction completion, all solvents are removed under reduced pressure and the residue is purified by silica gel column chromatography to yield compound **L.**

### Trimacrocyclic compound (M)

Trimacrocyclic compound **(L)** is dissolved in a 50% solution of 12 N HCl in acetic acid. The solution is stirred at ambient temperature for two days. Upon reaction completion, solids are filtered, and the filtrate is concentrated under reduced pressure to yield compound **M.**

### Example 2

### Synthesis of a protein-conjugated 1,2-HOPO trimacrocyclic chelator

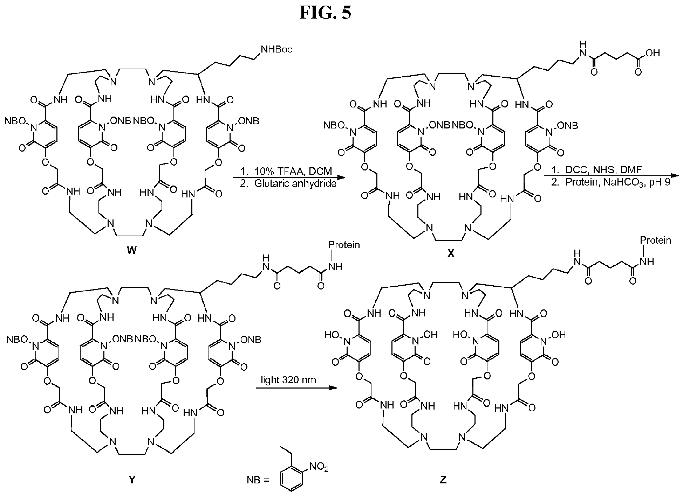

Figure 5 shows one possible multistep synthetic route for conjugating a targeting moiety to a chelator.

### Trimacrocyclic compound (W)

Trimacrocyclic compound **(W)** is prepared as described above for compound **L,** except that ortho-nitrobenzyl bromide (Aldrich Chemicals) is substituted for benzyl chloride in the synthesis.

### Trimacrocyclic compound (X)

Trimacrocyclic compound **(W)** is dissolved in a 10% solution of trifluoroacetic acid in dichloromethane. The solution is stirred at ice bath temperature for about four hours. Upon reaction completion, the solution is concentrated under reduced pressure. The residue is dissolved in dimethylformamide, diisopropylethylamine (3 molar equivalents) and glutaric anhydride (2 molar equivalents) is added, and the reaction is monitored by HPLC. Upon reaction completion, the reaction is neutralized with acetic acid, solvent is removed under reduced pressure, the residue is dissolved in a minimum amount of dimethylformamide, and this solution is added to diethyl ether. The resulting precipitate is filtered and dried in vacuo to yield compound **X.**

### Trimacrocyclic compound protein conjugate (Y)

Trimacrocyclic compound **(X)** is dissolved in anhydrous dimethylformamide. N-Hydroxysuccinimide (1.5 molar equivalents) and dicyclohexylcarbodiimide (3 molar equivalents is added and the solution is stirred for several hours. The resulting solution is added to a solution of protein (0.1 - 0.5 molar equivalents) in 0.4 M NaHCO₃ buffer, pH 9.0 and the resulting solution is mixed for several hours. The resulting protein conjugate is separated from any unreacted trimacrocyclic compound and buffer-exchanged into 0.1M TRIS, pH 7.0 using a size exclusion column or buffer exchanged to yield purified protein conjugate **Y.**

### Trimacrocyclic compound protein conjugate (Z)

Trimacrocyclic compound protein conjugate **(Y)** is irradiated at 320 nm for 10-30 minutes using a UV lamp. The resulting protein conjugate is separated from any ortho-nitrosobenzaldehyde by-product and buffer-exchanged into fresh 0.1M TRIS, pH 7.0 using a size exclusion column to yield purified protein conjugate **Z.**

### Example 3

### Crystal structure of Me4BH(2,2)IAM

The raw Me₄BH(2,2)IAM obtained from flash silica column purification is a mixture of two components, which show two discrete spots of silica TLC plate. One component with higher Rf was separated, and X-ray quality crystals were obtained by vapor diffusion of ether into the methanol solution of Me₄BH(2,2)IAM.

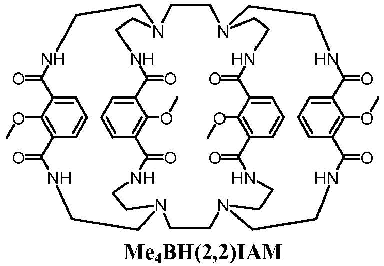

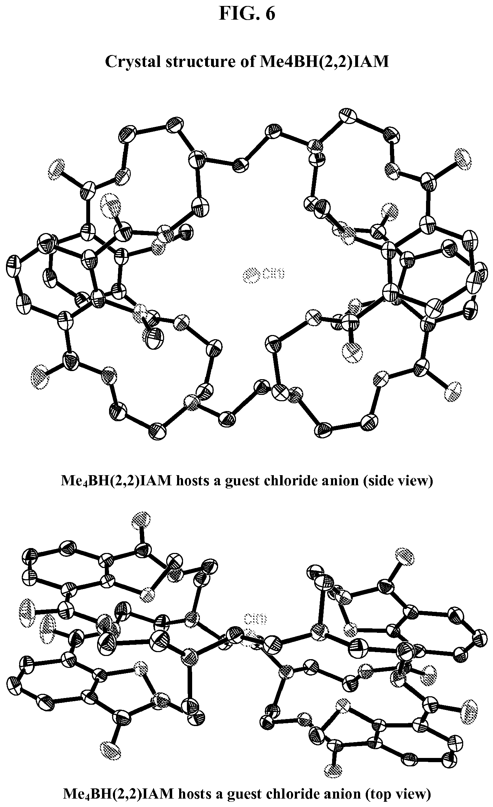

The crystal structure shown in Figure 6 reveals that this macrocycle hosts a chloride anion guest in the center of its pocket. The chloride anion might have been introduced in the process of preparation of this macrocycle.

### Example 4

### Crystal structure of H(2,2)-1,2-HOPO complexes

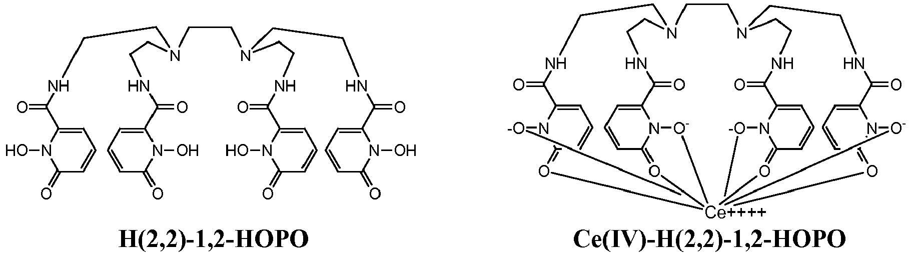

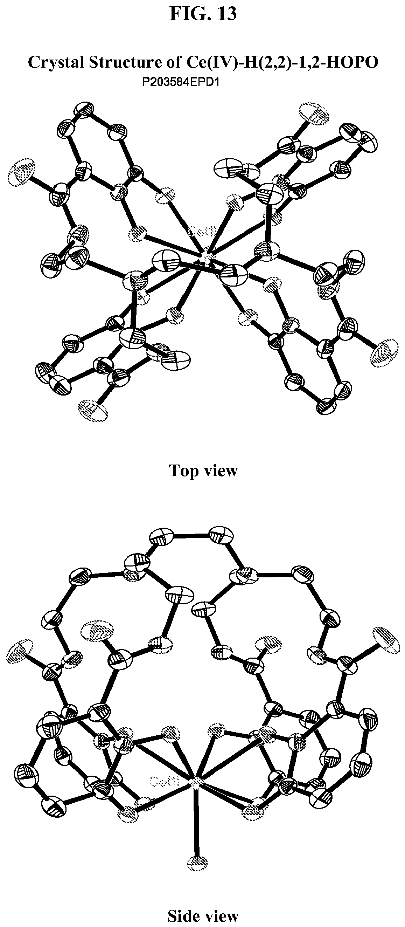

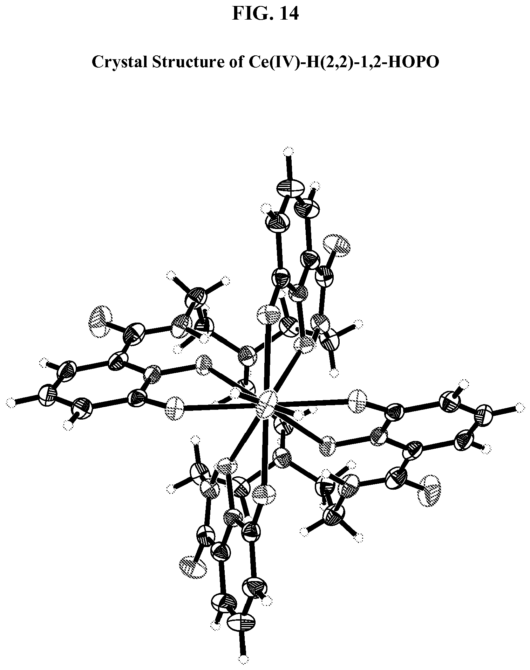

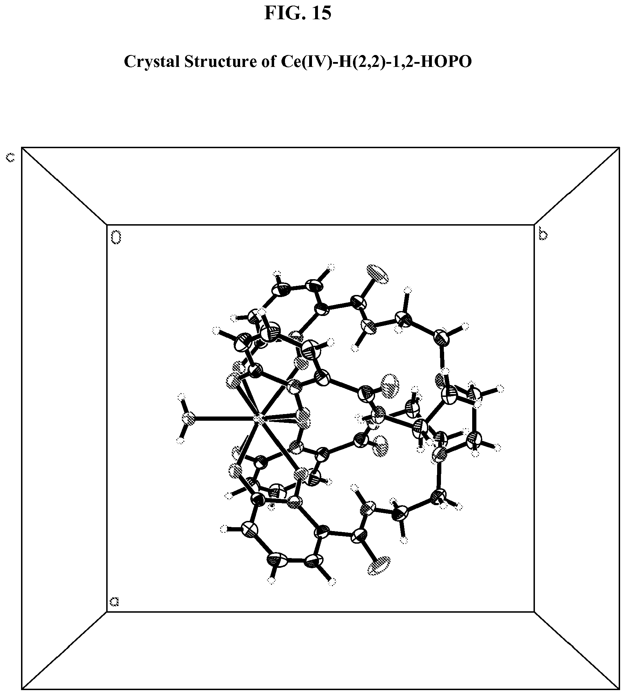

H(2,2)-1,2-HOPO is one of the most powerful octadentate ligands developed for sequestering lanthanide and actinide metal ions. It has been proved that H(2,2)-1,2-HOPO has strong affinity towards actinide and lanthanide ions, but there is no report on the crystal structure of its metal complexes. Recently, we prepared Ce(IV)-H(2,2)-1,2-HOPO complex by mixing methanol solutions of equivalent H(2,2)-1,2-HOPO and Ce(acac)₄. X-ray quality crystals was obtained by diffusion of diethyl ether into the above methanol solution. Figures 13-15 show the crystal structure of Ce(IV)-H(2,2)-1,2-HOPO.

### Example 5

### Mass spectrometry of Lumi4^{®}-NH₂ metal ion complexes

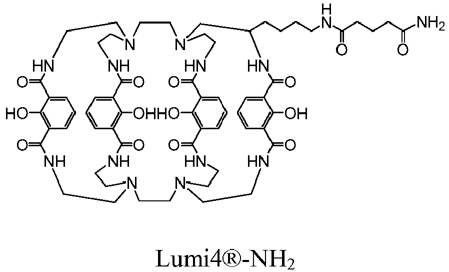

Complexes of Lumi4^{®}-NH₂ and the following cations (Fe(III), Ga(III), Y(III), Zr(IV), In(III), Nd(III), Sm(III), Eu(III), Gd(III), Dy(III), Ho(III), Er(III), Yb(III), Lu(III) and Th(IV) were prepared. All of these cations have isotopes which have been or are currently utilized for either radiotherapy or radioimaging. Anderson, C. J.; Welch, M. J. Chem. Rev. 1999, 99: 2219-2234. Complexes using the aforementioned cations were prepared using the following method. A solution of Lumi4^{®}-NH₂ was prepared in dry HPLC grade methanol at a concentration of 100 µM based on the percent weight of this lot (RCG23-DO2). Solutions of the cations were prepared at millimolar concentrations (10-100 mM) in either water or dry HPLC grade methanol. To each solution of Lumi4^{©}-NH₂ (50 nmol) a 1.01 equivalent of each metal cation solution (-50.5 nmol) was added, mixed and equilibrated for 10 min followed by addition of a drop of dry pyridine. All solutions were lyophilized under high vacuum. The resulting powders were submitted for electrospray ionization-mass spectroscopy (ESI-MS) at the UC Berkeley QB3/Chemistry Mass Spectrometry Facility. The Lumi4^{®}-NH₂ Zr(IV) and Th(IV) complexes were analyzed in ESI-MS positive mode (MH⁺). The remaining Lumi4^{®}-NH₂ complexes were analyzed by ESI-MS in negative mode (M⁻).

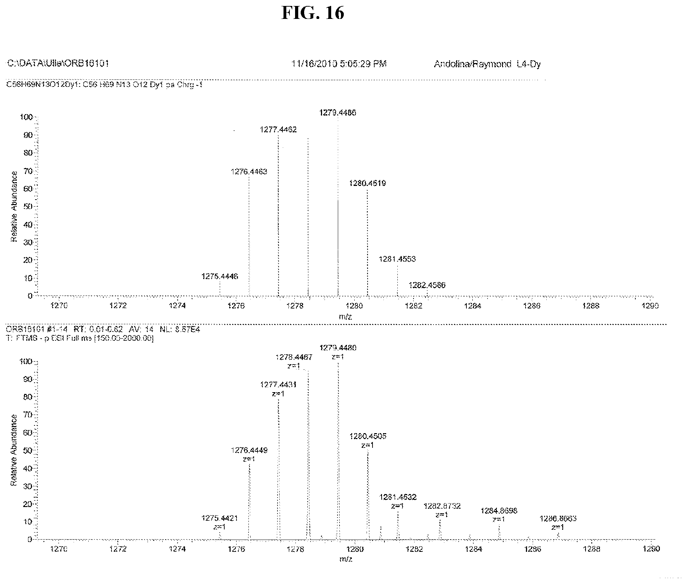

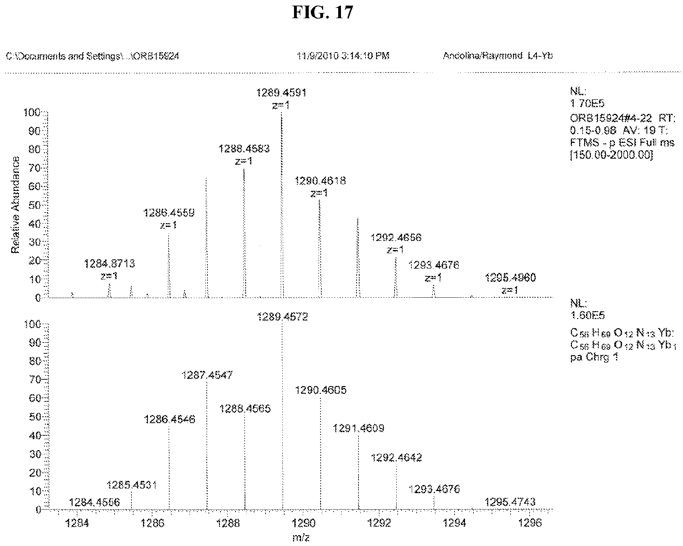

In Figures 16 and 17, representative ESI-MS spectra for two of the metals show the comparison of the experimental data (top) verses the calculated isotopic pattern (bottom) for each complex. These spectra, as well as others not shown here, verify that these complexes have been made. However, some spectra indicate interfering compounds which result in more peaks than predicted. These interfering species may be a background artifact (they change based on the day of analysis) this is a common occurrence (memory effects and ion suppression) with negative mode ESI-MS analysis. Table 1 summarizes the collected ESI-MS spectra, comparing the experimental, predicted predominant isotopes and the difference of these two values for each Lumi4^{®}-NH₂ complex. All values given are high resolution values within 2 ppm.

**Table 1. Comparison of the calculated most abundant peak of each Lumiphore metal cation complex, most abundant Lumiphore metal complex peak of the high resolution ESI-MS spectra (either M⁻ or MH⁺) and the difference between the two.**

| **M-L4** | **Calculated (m/z)** | **Found (m/z)** | **Difference (m/z)** |
|---|---|---|---|
| Fe(III) | 1171.4538 | 1171.4527 | 0.0011 |
| Ga(III) | 1184.4444 | 1184.4452 | -0.0008 |
| Y(III) | 1204.4247 | 1204.4252 | -0.0005 |
| Zr(IV) | 1206.4236 | 1206.4294 | -0.0058 |
| In(III) | 1230.4227 | 1230.4228 | -0.0001 |
| Nd(III) | 1257.4266 | 1257.4271 | -0.0005 |
| Sm(III) | 1267.4386 | 1267.4391 | -0.0005 |
| Eu(III) | 1268.4401 | 1268.4406 | -0.0005 |
| Gd(III) | 1273.4430 | 1273.4436 | -0.0006 |
| Dy(III) | 1279.4480 | 1279.4486 | -0.0006 |
| Ho(III) | 1280.4492 | 1280.4486 | 0.0006 |
| Er(III) | 1281.4492 | 1281.4497 | -0.0005 |
| Yb(III) | 1289.4577 | 1289.4591 | -0.0014 |
| Lu(III) | 1290.4596 | 1290.4602 | -0.0006 |
| Th(IV) | 1348.5569 | 1348.5628 | -0.0059 |

### Example 6

### Luminescence of Lumi4^{®}-NH₂ metal ion complexes

In addition, photoluminescent emission spectra and properties have been included for Lumiphore^{®}-NH₂ Eu(III) and Dy(III) complexes at a concentration of 77 µM in 0.1 M TRIS buffer pH = 7.4. The luminescent spectra collect are also an indication of complex formation. Excitation (and subsequent emission of the Ln(III)) under these conditions would be difficult to detect unless the Ln(III) was fully encapsulated into the chelator/chromophore (Lumiphore^{®}-NH₂).

**Table 2. Ln- Lumiphore^{®} photophysical measurements.**

| | Ln(III) | *Φ*_{Ln} | *τ* (ms) |
|---|---|---|---|
| Tb-Lumi4^{®} | Tb | 0.600 | 2.670 |
| Eu-Lumi4 | Eu | 0.002 | 0.575 |
| Dy-Lumi4 | Dy | 0.015 | < 0.020 |

### Example 7

### Kinetic data for Tb-Lumi4^{®}-NH₂

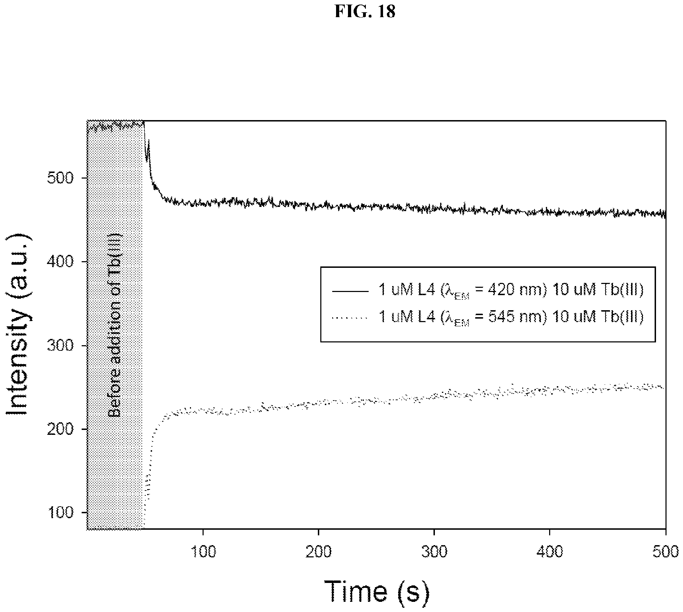

Kinetic association luminescent measurements were performed with the intention of determining the rate of complexation of Tb-Lumi4^{®}-NH₂ and estimated the rate of formation of Zr(IV)-Lumi4^{®}-NH₂. Measurements were made under the following conditions: 1 µM (3 nmol) Lumiphore^{®}-NH₂ in 3.00 mL 20.0 mM HEPES buffer *I* = 0.10 M NaCl. To this solution, concentrated aqueous (3.00 mM) Tb(III) was added to collect data under the following concentrations 10, 20, 30, 40, 50 and 100 µM Tb(III) to 1 µM Lumi4^{®}-NH₂. The resulting curves depicted multiple events (4-5) (Figure 18). Nevertheless, the quenching of the ligand fluorescence and the increase of Tb(III) luminescence were monitored at 420 and 545 nm respectively using a 20 nm bandpass, excitation at 340 nm (10 nm bandpass) using a Carey Eclipse Fluorometer (Varian, Inc.). The intensity data was collected in 0.5 s intervals with an integration time of 0.1 s. The experiment was started and the Tb(III) aliquot was quickly added and thoroughly mixed into the 3.00 mL Lumi4^{®}-NH₂ solution. The overall rate constant for formation was estimated to be < 0.1 s⁻¹ under these conditions 1 µM Lumi4^{®}-NH₂ to 10 µM Tb(III).

### Example 8

### Gel electrophoresis assays showing complexation of an oligonucleotide-Lumi4 chelate conjugate with various metal cations

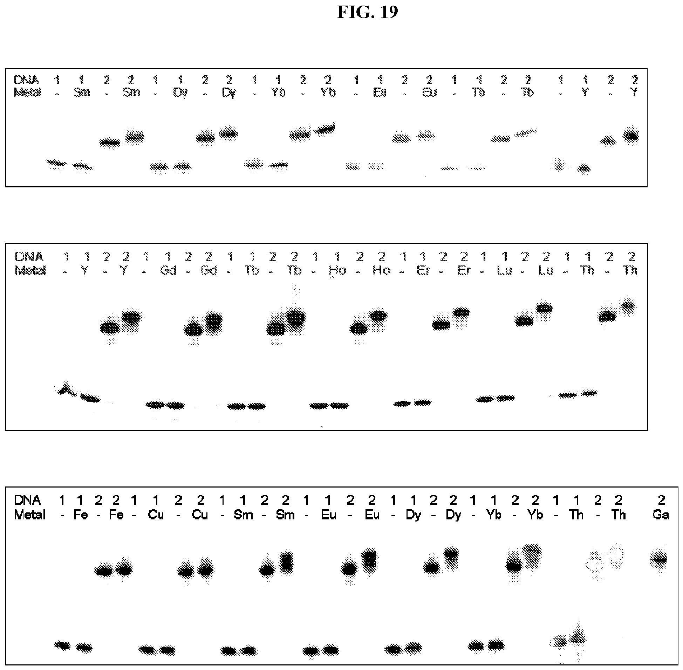

Lumi4^{®} is an isophthalamide class macrocyclic chelate that selectively coordinates to metal cations including those of the lanthanide series. For use in certain applications, such as acting as a bifunctional chelating agent to attach a radioisotope to a site-directing molecule, it is necessary that the chelate be able to coordinate to the metal ion of interest in a kinetically facile and thermodynamically stable manner. To demonstrate the utility of Lumi4^{®} for this type of application, the ability of Lumi4^{®} to coordinate to metal cations following conjugation with a site-directing molecule was assessed using a gel electrophoresis assay (Figure 19). In this experiment, a conjugate of Lumi4^{®} with a small (18 base length) DNA oligomer was treated with a solution containing a metal cation. The electrophoretic mobility of the conjugate on a polyacrylamide gel was then compared with that of the conjugate which was not exposed to the solution of metal cation. Metal complexation in this format is indicated by a gel electrophoresis mobility shift, such that the heavier and more positively charged species formed upon metal complexation migrates more slowly. To provide an additional comparison, the gel electrophoretic mobility of the DNA oligomer that was not conjugated to Lumi4^{®} was measured with and without exposure to the metal cation solution under identical conditions.

In particular, a solution of DNA oligomer (6 µL, 5 µM, all concentrations final) was mixed with a solution of metal cation (2 µL, 250 µM) or an equal volume of water. The solution was incubated at 55 °C for five minutes, whereupon the solutions were allowed to cool to ambient temperature and a solution of 50% formamide (7 µL) was added. The resulting solution was again heated to 55 °C for five minutes and then cooled to -20 °C briefly. The solution was then applied to a 20% polyacrylamide gel containing 8 M urea. Gel electrophoresis was conducted for about 2 hours using a commercial running buffer (Ambion AM9863) containing 89 mM tris(hydroxymethyl)aminomethane (TRIS), 89 mM borate, and 2 mM ethylenediaminetetraacetic acid (EDTA). Upon the completion of electrophoresis, the gel was removed from the glass plates and soaked in a 50% formamide solution containing 12.5 mg/mL Stains-All^{®} (Sigma Chemicals). After staining, the gel was destained in de-ionized water for 2 hours and imaged using a commercial scanner (HP Officejet^{®} J5750).

Inspection of the gels indicates that the oligonucleotide-Lumi4® conjugate migrates more slowly following treatment with the metal cation solution. In contrast, the gel mobility of the unmodified oligonucleotide is unaffected by metal cation treatment. These data indicate that Lumi4® chelate, when conjugated to a DNA oligomer, coordinates with facility to the metal cations tested and forms a stable complex even upon electrophoresis in the presence of the competing chelate EDTA. In summary, our findings suggest that Lumi4® when conjugated to a site-directing group coordinates readily with a variety of metal cations including those of the lanthanide series.

### Synthesis of oligonucleotide-Lumi4® conjugate (2)

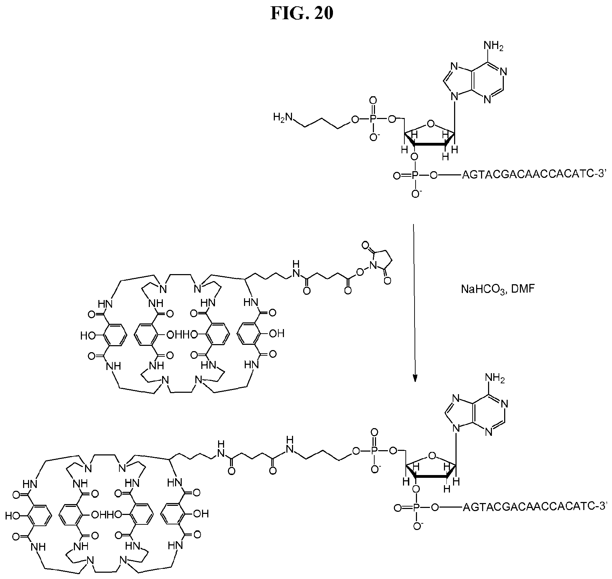

A DNA 18-base oligonucleotide (1) with the sequence 5'-AAGGTCATCCATGACAAC -3' was purchased commercially (Eurogentec, Inc., Seraing, Belgium) and purified using reverse-phase HPLC. The oligonucleotide was modified during synthesis to possess an aminopropyl group attached at the 5'-terminus via a phosphodiester linkage. A solution of DNA oligomer in water (75 µL, 50 nmol) was diluted with sodium bicarbonate buffer (0.8 M, 100 µL) in an eppendorf tube. A solution of Lumi4^{®}-N-hydroxysuccinimide (839 nmol) in anhydrous DMF (50 µL) was freshly prepared, added to the DNA oligomer and mixed at 800 rpm using a commercial device (Eppendorf Mixmate®) at ambient temperature for 10 hours. The eppendorf tube was centrifuged at 12,000 rpm for 10 minutes, and the supernatant decanted to a fresh eppendorf tube. The pellet was washed with water (75 µL), centrifuged as above, and the supernatant decanted. A solution (34 µL) of glycogen (350 µg/mL) in 3M sodium acetate, pH 5.2 was added to the combined supernatants. The solution was vortexed, absolute ethanol (1 mL) was added, and the tube was stored at -20 °C for three hours. The eppendorf tube was centrifuged at 12,000 rpm for 30 minutes, the supernatant decanted, and the resulting pellet was washed with cold, 70% aqueous ethanol (1 mL). The supernatant was decanted, and the pellet was allowed to dry open to the air. The pellet was dissolved in sterile water (50 µL), and an aliquot (5 µL) was removed to quantify by UV-visible absorbance using the extinction coefficient at 260 nm of 181,600 M⁻¹ cm⁻¹. The resulting stock was found to have a concentration of 730 µM (yield 73%). There was greater than 95% conversion to conjugate, as estimated from analysis by 20% polyacrylamide gel electrophoresis. The conjugate was used without further purification. Figure 20 shows a scheme for the synthesis of the oligonucleotide-Lumi4® conjugate.

### Preparation of metal ion stocks

In general, the chloride salts of metal cations were dissolved in 50 mM sodium citrate, pH 5, to provide primary stocks of 25 mM cation. These stocks were diluted to 2.5 mM in sterile water. In the case of Th(IV) nitrate, a 25 mM stock was prepared in methanol, and this was diluted to 2.5 mM using additional methanol. For copper and gallium, Cu(II) acetate and Ga(III) nitrate salts were used.

The articles "a," "an" and "the" as used herein do not exclude a plural number of the referent, unless context clearly dictates otherwise. The conjunction "or" is not mutually exclusive, unless context clearly dictates otherwise. The term "include" is used to refer to non-exhaustive examples.

All references, publications, patent applications, issued patents, accession records and databases cited herein, including in any appendices, are incorporated by reference in their entirety for all purposes.
The present application and invention further includes the subject matter of the following numbered clauses:
1. A complex comprising (a) a radionuclide and (b) a macrocycle comprising (i) a plurality of chelating moieties, (ii) a linker, (iii) a first scaffold moiety and (iv) a second scaffold moiety, wherein each of the chelating moieties is attached to the first scaffold moiety and the second scaffold moiety.
2. The complex of clause 1 wherein the macrocycle comprises 3, 4 or 5 chelating moieties.
3. The complex of any preceding clause wherein the plurality of chelating moieties comprises a plurality of oxygen donors and the radionuclide is chelated to the macrocycle via at least one of the oxygen donors.
4. The complex of any preceding clause wherein the macrocycle comprises a plurality of oxygen donors and the radionuclide is chelated to the macrocycle via a plurality or all of the oxygen donors.
5. The complex of any preceding clause wherein the chelating moieties are independently selected from 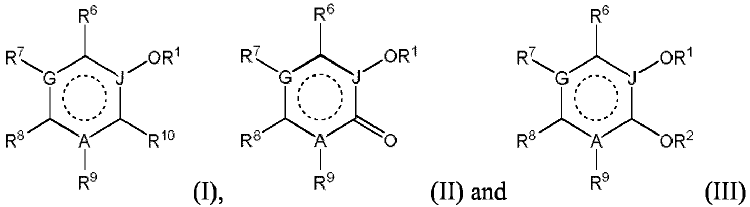
   wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, -NO₂,
   R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl;
   R¹⁷ and R¹⁸, together with the atoms to which they are attached, are optionally joined to form a 5-, 6- or 7-membered ring;
   at least two of R⁶, R⁷, R⁸, R⁹ and R¹⁰ are optionally joined to form a ring system which is a member selected from substituted or unsubstituted cycloalkyl, substituted or unsubstituted heterocycloalkyl, substituted or unsubstituted aryl and substituted or unsubstituted heteroaryl;
   R¹ and R² are each independently selected from H and a negative charge;
   A, G and J are independently selected from carbon and nitrogen; and
   wherein one of R⁶ and R⁹ in (II) or (III) or one of R⁶ and R¹⁰ in (I) comprises a bond to the first scaffold moiety, with the other of R⁶ and R⁹ in (II) or (III) and the other of R⁶ and R¹⁰ in (I) comprising a bond to the second scaffold moiety.
6. The complex of clause 5 wherein if one of the chelating moieties has the structure 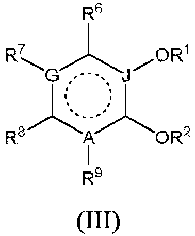 then all of the chelating moieties are not the same.
7. The complex of clause 6 wherein one of the chelating moieties has the structure 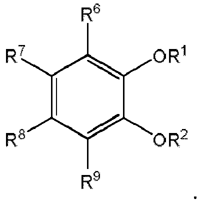
8. The complex of clause 5 wherein the chelating moieties all have the structure 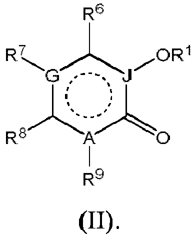
9. The complex of any of clauses 5 and 8 wherein, in structure (II), A is nitrogen and G and J are carbon.
10. The complex of any of clauses 5 and 8 wherein, in structure (II), J is nitrogen and A and G are carbon.
11. The complex of clause 5 wherein the chelating moieties all have the structure 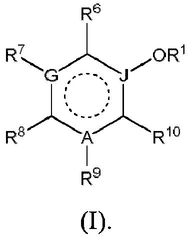
12. The complex of any of clauses 5 and 11 wherein R⁹ of 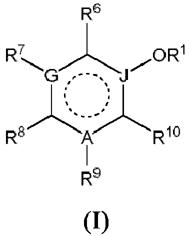 is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl.
13. The complex of any of clauses 5, 11 and 12 wherein R⁹ of 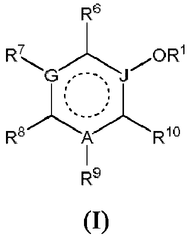 is H.
14. The complex of any of clauses 5 and 11-13 wherein, in structure (I), A, G and J are carbon.
15. A complex comprising (a) a radionuclide and (b) a chelator comprising (i) a plurality of chelating moieties and (ii) a first scaffold moiety, wherein each of the chelating moieties is attached to the first scaffold moiety and wherein each of the chelating moieties has a structure independently selected from 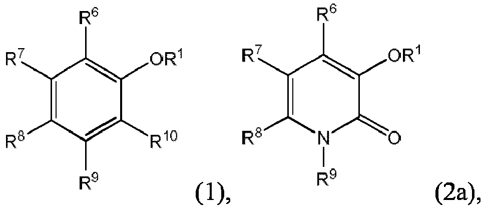 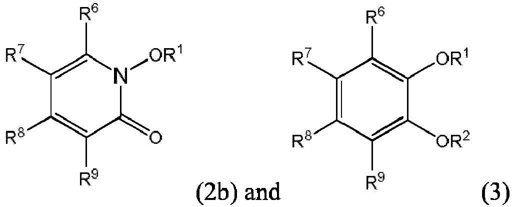
   wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸ and -NO₂,
   R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl;
   R¹⁷ and R¹⁸, together with the atoms to which they are attached, are optionally joined to form a 5-, 6- or 7-membered ring;
   at least two of R⁶, R⁷, R⁸, R⁹ and R¹⁰ are optionally joined to form a ring system selected from substituted or unsubstituted cycloalkyl, substituted or unsubstituted heterocycloalkyl, substituted or unsubstituted aryl and substituted or unsubstituted heteroaryl;
   R¹ and R² are each independently selected from H and a negative charge;
   one of R⁶ and R⁹ in (2a), (2b) and (3) and one of R⁶ and R¹⁰ in (1) comprises a bond to the first scaffold moiety,
   wherein at least one chelating moiety has the structure 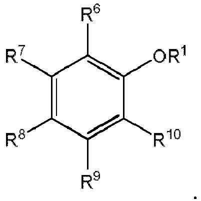
16. The complex of clause 15 wherein all the chelating moieties have the structure 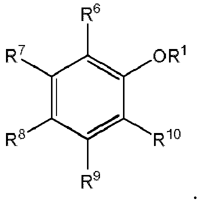
17. The complex of any of clauses 15 and 16 wherein if R⁶ is attached to the first scaffold moiety, then R⁹ in (2a), (2b) and (3) or R¹⁰ in (1) is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl; and if R⁹ in (2a), (2b) and (3) or R¹⁰ in (1) is attached to the first scaffold moiety, then R⁶ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl.
18. The complex of any of clauses 15-17 wherein if R⁶ is attached to the first scaffold moiety, then R⁹ in (2a), (2b) and (3) or R¹⁰ in (1) is H; and if R⁹ in (2a), (2b) and (3) or R¹⁰ in (1) is attached to the first scaffold moiety, then R⁶ is H.
19. The complex of any of clauses 15-18 wherein R¹⁰ is -C(O)NR¹⁷R¹⁸.
20. The complex of clause 19 wherein R¹⁷ and R¹⁸ are each independently selected from H, substituted or unsubstituted alkyl and substituted or unsubstituted heteroalkyl.
21. The complex of any of clauses 15-20 further comprising a linker.
22. A complex comprising (a) a radionuclide and (b) a first chelator comprising (i) a plurality of chelating moieties, (ii) a linker and (iii) a first scaffold moiety, wherein each of the chelating moieties is attached to the first scaffold moiety and wherein each of the chelating moieties has a structure independently selected from: 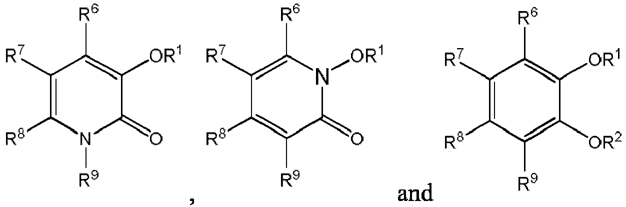
   wherein each R⁶, R⁷, R⁸, R⁹ and R¹⁰ in each chelating moiety are independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, -NO₂,
   R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl;
   R¹⁷ and R¹⁸, together with the atoms to which they are attached, are optionally joined to form a 5-, 6- or 7-membered ring;
   at least two of R⁶, R⁷, R⁸, R⁹ and R¹⁰ are optionally joined to form a ring system selected from substituted or unsubstituted cycloalkyl, substituted or unsubstituted heterocycloalkyl, substituted or unsubstituted aryl and substituted or unsubstituted heteroaryl;
   R¹ and R² are each independently selected from H and a negative charge;
   one of R⁶ and R⁹ in each chelating moiety comprises a bond to the first scaffold moiety.
23. The complex of clause 22 wherein the chelating moieties are selected from 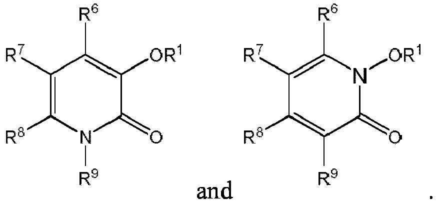
24. The complex of any of clauses 22 and 23 wherein if R⁶ is attached to the first scaffold moiety, then R⁹ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl; and if R⁹ is attached to the first scaffold moiety, then R⁶ is selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl.
25. The complex of any of clauses 22-24 wherein if R⁶ is attached to the first scaffold moiety, then R⁹ is H; and if R⁹ is attached to the first scaffold moiety, then R⁶ is H.
26. The complex of any of clauses 22-25 further comprising a second chelator having the same structure as the first chelator.
27. The complex of any of clauses 22-26 wherein the chelating moieties are not all the same.
28. The complex of any of clauses 1-14 wherein the second scaffold moiety is substituted heteroalkyl.
29. The complex of any of clauses 1-14 and 28 wherein the second scaffold moiety has the structure 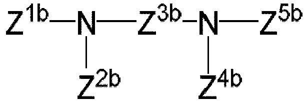
   wherein Z^{1b}, Z^{2b}, Z^{3b}, Z^{4b} and Z^{5b} are independently selected from substituted or unsubstituted alkyl and substituted or unsubstituted heteroalkyl; and
   each of Z^{1b}, Z^{2b}, Z^{4b} and Z^{5b} comprises a bond to one of the chelating moieties.
30. The complex of clause 29 wherein Z^{3b} is substituted or unsubstituted (C₁, C₂, C₃, C₄, C₅ or C₆) alkyl.
31. The complex of clause 29 wherein Z^{3b} is substituted or unsubstituted -(CH₂)ₘ(CH₂CH₂O)ₙ(CH₂)ₚ-, wherein m, n and p are integers independently selected from 1, 2, 3, 4, 5 and 6.
32. The complex of clause 29 wherein Z^{3b} is ethyl substituted by =O.
33. The complex of clause 29 wherein Z^{3b} is ethyl.
34. The complex of any of clauses 29-33 wherein Z^{1b}, Z^{2b}, Z^{4b} and Z^{5b} have a structure selected from Z'R^{20b}N(H)C(O)Z", Z'R^{20b}N(H)C(O)R^{21b}Z" and Z'R^{21b}Z"
   wherein Z' is a bond to the second scaffold moiety,
   Z" is a bond to one of the plurality of chelating moieties,
   R^{20b} is selected from substituted or unsubstituted alkyl and substituted or unsubstituted heteroalkyl and,
   R^{21b} is selected from substituted or unsubstituted alkyl and substituted or unsubstituted heteroalkyl.
35. The complex of clause 34 wherein R^{20b} is selected from substituted or unsubstituted (C₁, C₂, C₃, C₄, C₅ or C₆) alkyl and substituted or unsubstituted (C₁, C₂, C₃, C₄, C₅ or C₆) heteroalkyl.
36. The complex of clause 34 wherein R^{20b} is selected from substituted or unsubstituted ethyl.
37. The complex of any of clauses 34-36 wherein R^{21b} is selected from substituted or unsubstituted -(CH₂)_{w}O- wherein w is selected from 1, 2, 3, 4, 5 and 6.
38. The complex of clause 37 wherein R^{21b} wherein w is 1 or 3.
39. The complex of any of clauses 29-38 wherein at least one of Z^{1b}, Z^{2b}, Z^{3b} Z^{4b} and Z^{5b} is substituted by a linker.
40. The complex of any preceding clause wherein the first scaffold moiety is substituted heteroalkyl.
41. The complex of any preceding clause wherein the first scaffold moiety has the structure 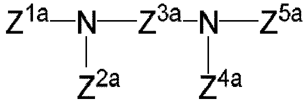
   wherein Z^{1a}, Z^{2a}, Z^{3a}, Z^{4a} and Z^{5a} are independently selected from substituted or unsubstituted alkyl and substituted or unsubstituted heteroalkyl; and
   Z^{1a}, Z^{2a}, Z^{4a} and Z^{5a} comprise a bond to one of the chelating moieties.
42. The complex of clause 41 wherein Z^{3a} is substituted or unsubstituted (C₁, C₂, C₃, C₄, C₅ or C₆) alkyl.
43. The complex of clause 41 wherein Z^{3a} is substituted or unsubstituted -(CH₂)ₘ(CH₂CH₂O)ₙ(CH₂)ₚ-, wherein m, n and p are integers independently selected from 1, 2, 3, 4, 5 and 6.
44. The complex of clause 41 wherein Z^{3a} is ethyl substituted by =O.
45. The complex of clause 41 wherein Z^{3a} is ethyl.
46. The complex of any of clauses 41-45 wherein Z^{1a}, Z^{2a}, Z^{4a} and Z^{5a} have a structure selected from Z'R^{20a}N(H)C(O)Z", Z'R^{20a}N(H)C(O)R^{21a}Z" and Z'R^{21a}Z" whe
   rein Z' is a bond to the second scaffold moiety,
   Z" is a bond to one of the plurality of chelating moieties,
   R^{20a} is selected from substituted or unsubstituted alkyl and substituted or unsubstituted heteroalkyl and,
   R^{21a} is selected from substituted or unsubstituted alkyl and substituted or unsubstituted heteroalkyl.
47. The complex of clause 46 wherein R^{20a} is selected from substituted or unsubstituted (C₁, C₂, C₃, C₄, C₅ or C₆) alkyl and substituted or unsubstituted (C₁, C₂, C₃, C₄, C₅ or C₆) heteroalkyl.
48. The complex of clause 46 wherein R^{20a} is selected from substituted or unsubstituted ethyl.
49. The complex of any of clauses 46-48 wherein R^{21a} is selected from substituted or unsubstituted -(CH₂)_{w}O- wherein w is selected from 1, 2, 3, 4, 5 and 6.
50. The complex of clause 49 wherein R^{21a} wherein w is 1 or 3.
51. The complex of any of clauses 46-50 wherein at least one of Z^{1a}, Z^{2a}, Z^{3a}, Z^{4a} and Z^{5a} is substituted by a linker.
52. The complex of any of clauses 1-40 wherein the first scaffold moiety has the structure 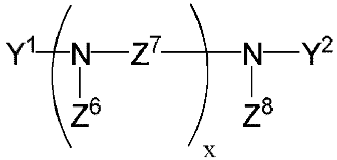
   wherein x is selected from 1, 2, 3 and 4;
   Y¹ and Y² are independently selected from H, substituted or unsubstituted alkyl and substituted or unsubstituted heteroalkyl;
   Z⁷ is selected from substituted or unsubstituted alkyl and substituted or unsubstituted heteroalkyl;
   Z⁶ and Z⁸ are independently selected from -C(O)-, substituted or unsubstituted alkyl, and substituted or unsubstituted heteroalkyl; and
   each of Z⁶ and Z⁸ comprises a bond to one of the chelating moieties
53. The complex of clause 52 wherein each Z⁷ is independently substituted or unsubstituted (C₁, C₂, C₃, C₄, C₅ or C₆) alkyl.
54. The complex of clause 52 wherein each Z⁷ is independently substituted or unsubstituted propyl or butyl.
55. The complex of clause 52 wherein each Z⁷ is independently substituted or unsubstituted heteroalkyl.
56. The complex of clause 52 wherein each Z⁷ is independently substituted or unsubstituted -(CH₂)ₘ(CH₂CH₂O)ₙ(CH₂)ₚ-, wherein m, n and p are integers independently selected from 1, 2, 3, 4, 5 and 6.
57. The complex of clause 52 wherein Z⁷ is substituted or unsubstituted -(CH₂)₂O(CH₂)₂-.
58. The complex of any of clauses 52-57 wherein Z⁶ and Z⁸ are -C(O)-.
59. The complex of any of clauses 52-58 wherein at least one Z⁷ is substituted by a linker.
60. The complex of any of clauses 1-14, 21-27, 39, 51 and 59 wherein the linker is selected from a functionalized linker and a linker to a targeting moiety.
61. The complex of clause 60 wherein the linker has the structure -L¹¹-X, wherein L¹¹ is selected from a bond, acyl, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; and X is a reactive functional group or the targeting moiety.
62. The complex of clause 61 wherein L¹¹ is selected from substituted or unsubstituted alkyl and substituted or unsubstituted heteroalkyl.
63. The complex of any of clauses 61 and 62 wherein X is selected from -NN₂ and -CO(O)H.
64. The complex of clause 61 wherein -L¹¹-X is selected from 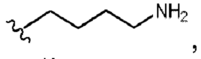 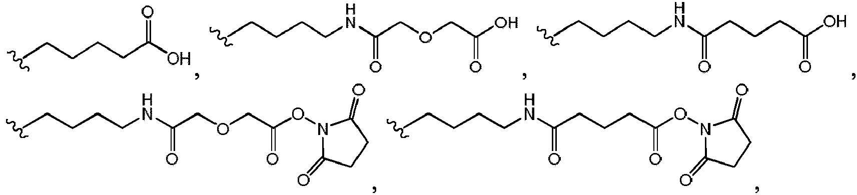 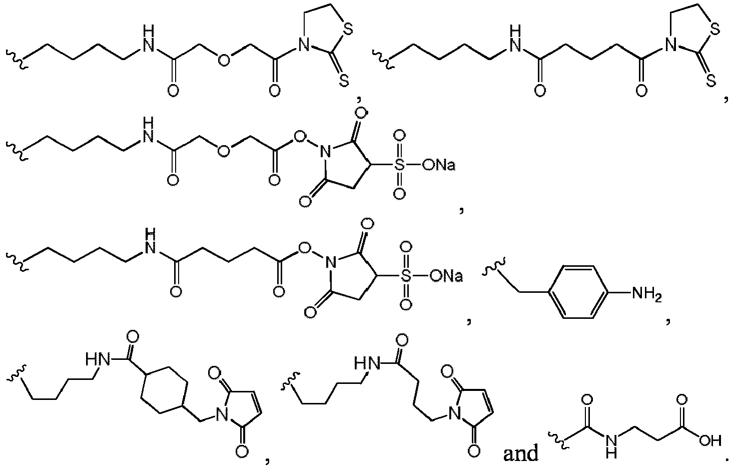
65. The complex of any of clauses 60-62 wherein the linker is a linker to a targeting moiety.
66. The complex of clause 65 wherein targeting moiety is selected from a peptide, a polypeptide, a nucleic acid, an oligonucleotide, a lipid, a polysaccharide, a small molecule, a cofactor and a hormone.
67. The complex of clause 66 wherein the targeting moiety is an antibody or antibody fragment.
68. The complex of any of clauses 5-67 wherein R⁷ and R⁸ are selected from H, halogen, alkyl, haloalkyl, heteroalkyl, aryl, heteroaryl, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl.
69. The complex of any of clauses 5-67 wherein R⁷ and R⁸ are H.
70. The complex of any of clauses 5-69 wherein R¹⁷ and R¹⁸ are selected from H and (C₁, C₂, C₃, C₄, C₅ or C₆) alkyl.
71. The complex of any preceding clause wherein the radionuclide is an emitter of a gamma ray or a particle selected from an alpha particle, an electron and a positron.
72. The complex of any preceding clause wherein the radionuclide is selected from an actinide and a lanthanide.
73. The complex of any of clauses 1-71 wherein the radionuclide is an ion of an atom selected from U, Pu, Fe, Cu, Ce, Nd, Eu, Sm, Gd, Tb, Dy, Ho, Er, Yb, Lu, Y, Th, Zr, In, Ga, Bi, Ra and Ac.
74. The complex of any of clauses 1-71 wherein the radionuclide is selected from Ra-223, Bi-213, Ac-225 and U-230.
75. The complex of any of clauses 1-71 wherein the radionuclide is an isotope of Th.
76. The complex of clause 75 wherein the radionuclide is selected from Th-227 and Th-232.
77. The complex of any preceding clause wherein the radionuclide is a 2⁺, 3⁺ or 4⁺ ion.
78. The complex of any preceding clause wherein the radionuclide is a 3⁺ ion.
79. A method of treating a disease in an animal comprising administering the complex of any preceding clause to the animal, whereby the disease is ameliorated or eliminated.
80. A method of diagnosing a disease in an animal comprising (a) administering the complex of any of clauses 1-78 to the animal and (b) detecting the presence or absence of a signal emitted by the complex.
81. The method of clause 80 wherein the detecting step comprises obtaining an image based on the signal.
82. The method of any of clauses 79-81 wherein the disease is cancer.
83. The method of any of clauses 79-82 wherein the complex comprises a linker to a targeting moiety and the method further comprises localizing the complex to a targeting site in the animal by binding the targeting moiety to the targeting site.

## Claims

1. A complex comprising (a) a radionuclide and (b) a first chelator comprising (i) a plurality of chelating moieties, (ii) a linker, and (iii) a first scaffold moiety,
wherein each of said chelating moieties is attached to said first scaffold moiety and wherein each of said chelating moieties has a structure independently selected from: 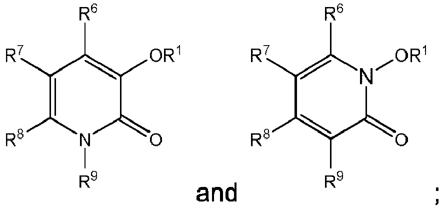
wherein each R⁶ is attached to said first scaffold moiety;
wherein each R⁷, R⁸ and R⁹ in each chelating moiety are independently selected from H, halogen, alkyl, haloalkyl, heteroalkyl, aryl, heteroaryl; -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸, wherein R¹⁷ and R¹⁸ are selected from H and alkyl; wherein each R¹ is H or a negative charge;
wherein said linker is -L¹¹-X,
wherein L¹¹ is selected from a bond, acyl, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl; and
wherein X is a reactive functional group or a targeting moiety,
wherein said reactive functional group is selected from olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitro, nitriles, mercaptans, sulfides, disulfides, sulfoxides, sulfones, sulfonic acids, sulfinic acids, acetals, ketals, anhydrides, sulfates, sulfenic acids isonitriles, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines, azides, azo compounds, azoxy compounds, nitroso compounds, maleimides, sulfonylhydrazide N-hydroxysuccinimide (NHS) esters, sulfo-NHS esters, imidoesters, acylhalides, arylazides, p-nitrophenyl esters, sulfonyl chlorides, thiazolides and carboxyl groups; and
wherein said targeting moiety is selected from a peptide, a polypeptide, a nucleic acid, an oligonucleotide, a carbohydrate, a lipid, a hormone, a growth factor, lectins, receptors, receptor ligands, cofactors, a polysaccharide and a small molecule;
wherein said first scaffold moiety has the structure: 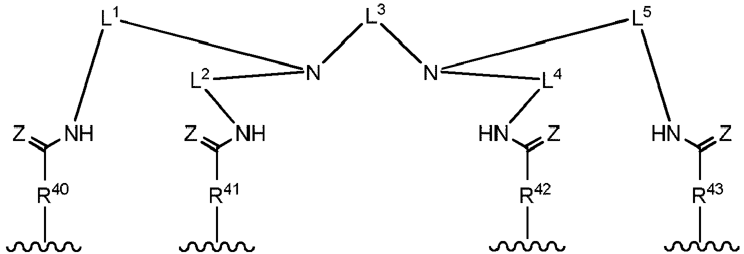
wherein each Z is independently selected from O and S;
wherein R⁴⁰, R⁴¹, R⁴² and R⁴³ are bonds;
wherein L³ is R³¹, and
R³¹ is (C₁, C₂, C₃, C₄, C₅ or C₆) alkyl substituted with said -L¹¹-X; and
wherein L¹, L², L⁴ and L⁵ are independently selected from substituted or unsubstituted (C₁, C₂, C₃, C₄, C₅ or C₆) alkyl.

2. The complex of claim **1,** wherein said radionuclide is thorium, optionally thorium-227 or thorium-232.

3. The complex of claim **1** or claim **2,** wherein each R⁷ and R⁸ are H.

4. The complex of any of claims **1** to **3,** wherein R⁹ is H or (C₁, C₂, C₃, C₄, C₅ or C₆) alkyl.

5. The complex of any of claims **1** to **3,** wherein R⁹ is methyl.

6. The complex of any of claims **1** to **5,** wherein said L¹¹ is selected from substituted alkyl, substituted heteroalkyl, substituted aryl, and substituted heteroaryl.

7. The complex of any of claims **1** to **6,** wherein said X is a targeting moiety.

8. The complex of claim **7,** wherein said targeting moiety is an antibody.

9. The complex of claim **8,** wherein said antibody is a full-length antibody or an antibody fragment.

10. The complex of any of claims **1** to **6,** wherein said X is a reactive functional group selected from the group consisting of: N-hydroxysuccinimide (NHS) esters, sulfo-NHS esters, maleimides and isothiocyanates.

11. The complex of any of claims **1** to **10,** wherein said R³¹ is C₃ or C₄ alkyl.

12. The complex of any of claims **1** to **11,** wherein said L¹, L², L⁴ and L⁵ are independently selected from substituted or unsubstituted ethyl.

13. The complex of any of claims **1** to **12,** wherein said Z is O.

14. The complex of any of claims **1** to **11,** wherein said first scaffold moiety has the structure:
